# EUROPEAN PATENT SPECIFICATION

(11) **EP 1 468 089 B1**
(45) Date of publication and mention of the grant of the patent: **08.04.2009**
(21) Application number: 03729485.7
(22) Date of filing: 20.01.2003
(51) Int. Cl.: C12N 15/10, C12Q 1/68

(54) **METHOD TO DETERMINE IN VIVO NUCLEIC ACID LEVELS**
VERFAHREN ZUR IN VIVO QUANTIFIZIERUNG VON NUKLEINSÄUREN
METHODE PERMETTANT DE DETERMINER LES TAUX D'ACIDE NUCLEIQUE IN VIVO

(30) Priority: 18.01.2002 EP 02447009
(43) Date of publication of application: 20.10.2004
(73) Proprietor: Université Libre de Bruxelles, 1070 Bruxelles (BE)
(72) Inventor: STORDEUR, Patrick, B-1190 Bruxelles (BE); GOLDMAN, Michel, B-1180 Bruxelles (BE)
(74) Representative: De Clercq, Ann G. Y.
(86) International application number: PCT/EP2003/000493
(87) International publication number: WO 2003/060119

(56) References cited:
- WO-A-02/00599
- WO-A-94/18156
- WO-A-94/28123
- WO-A-99/49083
- WO-A-99/57130
- WO-A-02/086113
- FR-A- 2 798 673
- US-A- 5 906 744
- DATABASE BIOSIS [Online] BIOSCIENCES INFORMATION SERVICE, PHILADELPHIA, PA, US; 16 November 2001 (2001-11-16) RAINEN LYNNE ET AL: "Stabilization of expression profiles of genes associated with the inflammatory response in post-phlebotomy whole blood using the PAXgeneTM blood RNA system" Database accession no. PREV200200151792 XP002260618 & BLOOD, vol. 98, no. 11 Part 2, 16 November 2001 (2001-11-16), pages 108b-109b, 43rd Annual Meeting of the American Society of Hematology, Part 2;Orlando, Florida, USA; December 07-11, 2001 ISSN: 0006-4971
- DATABASE BIOSIS [Online] BIOSCIENCES INFORMATION SERVICE, PHILADELPHIA, PA, US; 16 November 2001 (2001-11-16) MUELLER MARTIN C ET AL: "Improvement of molecular monitoring of residual disease in leukemias by bedside RNA stabilization with the PAXgene blood RNA system" Database accession no. PREV200200129905 XP002260619 & BLOOD, vol. 98, no. 11 Part 1, 16 November 2001 (2001-11-16), pages 111a-112a, 43rd Annual Meeting of the American Society of Hematology, Part 1;Orlando, Florida, USA; December 07-11, 2001 ISSN: 0006-4971
- "PAXgene Blood RNA Kit Handbook" February 2004 (2004-02), PREANALYTIX GMBH
- "PAXgene Blood RNA Kit Handbook" February 2004 (2004-02), PREANALYTIX GMBH

## Description

### Technical field

The present invention relates to a new nucleic acid analysis method in particular to determine the correct *in vivo* levels of low-level or unstable nucleic acid transcripts in whole blood.

### Background art

Deoxyribonucleic acid (DNA), and ribonucleic acid (RNA) are employed in a wide variety of research, medical, diagnostic and industrial processes. The variety of uses for extracted and purified DNA and RNA from disparate sources is rapidly increasing with the advent of biotechnology e.g. for the production of recombinant proteins.
Alternatively, nucleic acid sequences can be employed for diagnostic purposes. For example they can be used to detect the presence of a specific biological agent such as pathogens, viruses or to determine abnormal metabolic changes. With a biological agent is meant all types of agents carrying nucleic acids. Nucleic acid analysis may allow to identify genetic and familial disorders, genetic aberrations and allow to prove identity. Also cellular states (induction of genes, differentiation, etc.) can be identified by visualizing nucleic acid sequences.

In some cases only a qualitative analysis is necessary determining the absence or presence of a specific nucleic acid sequence and/or biological agent. In other cases, real transcript levels need to be determined. Indeed, certain diseases are characterized by the lowered or the increased level of gene expression; certain cell types can only be identified by evaluating the transcript content.

Until now, many tools are available enabling the person, skilled in the art, to perform an isolation of nucleic acids from different biological samples. The collection of a biological sample is the first step in many molecular assays used to study their nucleic acid content.

A major challenge in this type of testing, however, is the instability of RNA *in vitro* especially when the detection of low-level RNA or unstable RNA is aimed at. Even the degradation of only a small fraction of the RNA may change the interpretation of the *in vivo* levels. Some transcripts are known to be present at low copy in a cell; other transcripts have an "AU-rich" sequence in their 3' end promoting their fast degradation by endogenous RNAses. Studies have shown that RNA rapidly degrades significantly within hours after sample collection. Furthermore, certain species of RNA, through the process of gene induction, increase once the sample is collected. Both RNA degradation and *in vitro* gene induction can lead to an under- or over-estimation of the *in vivo* gene transcript number.

Until now, many methods exist to isolate RNA from biological samples. Some allow even the determination of low-level transcripts out of a pool of transcripts. Nevertheless, none of them provide the possibility to determine real *in vivo* levels. With 'real *in vivo* levels' is meant the level(s) of transcript(s) present in the biological agent at the time of the sampling. Storage of biological samples leads to incorrect mRNA levels. Indeed, in practice, the analysis of fresh sample is not feasible as the place of sampling and the place of RNA analysis is located differently.

WO 02/00599, WO 94/18156 and Rainen et al. Blood (2001, 98:108b-109b) indicate that nucleic acid isolation and stabilization may be performed for the detection of high level RNA.

Recently, PreAnalytiX (a joint venture between Becton Dickinson and Qiagen) has put its first product PAXgene^{™} Blood RNA System on the market. The PAXgene^{™} Blood RNA System (also referred to as the Qiagen method) is an integrated and standardized system for the collection and stabilization of whole blood specimens and isolation of cellular RNA. According to PreAnalytiX, in the PAXgene^{™} Blood RNA System, blood is collected directly into PAXgene^{™} Blood RNA Tubes and RNA is subsequently isolated using the PAXgene^{™} Blood RNA Kit.

The PAXgene^{™} Blood RNA Tube is a plastic, evacuated tube, for the collection of whole blood and stabilization of the cellular RNA profile. The tubes contain an additive (a proprietary blend of reagents) that stabilizes cellular RNA and may eliminate *ex vivo* induction of gene transcription and prevents the drastic changes in the cellular RNA expression profiles that normally take place *in vitro.* RNA is then isolated using silica-gel-membrane technology supplied in the PAXgene^{™} Blood RNA Kit. According to PreAnalytiX, the resulting RNA accurately represents the expression profile *in vivo* and is suitable for use in a range of downstream applications. According to the supplier, accurate quantification of gene transcripts is possible using this system. A major disadvantage of this PAXgene^{™} Blood RNA System is that respective PAXgene^{™} Blood RNA Tube needs to be combined with the PAXgene^{™} Blood RNA Kit (see instruction manual of the PAXgene^{™} Blood RNA Tubes). This obliged combination, however, limits further improvement of the system.

### Obiects of the invention

Although the PreanalytiX (or PAXgene^{™} Blood RNA) System points towards the fact that the PAXgene^{™} Blood RNA Tubes can only be combined with the PAXgene^{™} Blood RNA Kit, the present invention aims to improve the suggested system. In addition, the present invention aims to develop a new method allowing the characterization of real *in vivo* transcript levels of low-level or unstable transcripts.

These aims have been met by following embodiments.

The present invention relates to a method for the quantification of real *in vivo* levels of low-level or unstable RNA from a-wole blood comprising the steps of:
(a) collecting the whole blood in a tube comprising a compound inhibiting RNA degradation and/or gene induction,
(b) forming a precipitate comprising nucleic acids,
(c) separating said precipitate of step (b) from the supernatant,
(d) dissolving said precipitate of step (c) using a buffer, forming a suspension,
(e) isolating nucleic acids from said suspension of step (d) using an automated device,
(f) dispersing/distributing a reagent mix for RT-PCR using an automated device,
(g) dispersing/distributing the nucleic acids isolated in step (e) within the dispersed reagent mix of step (f) using an automated device, and,
(h) determining the *in vivo* levels of low-level OR unstable RNA using the nucleic acid/RT-PCR reagent mix of step (g) in an automated setup.

Inhibition of RNA degradation and/or gene induction at the moment of the whole blood sampling is crucial in order to retrieve a pool of RNAs which can be used to determine the *in vivo* transcript levels. It is true that cellular RNA can be purified using the PAXgene^{™} Blood RNA System; nevertheless, the present invention proves that real *in vivo* levels of low-level or unstable RVA from whole blood can not be measured using this system 'as such' (see example 2 of).

The present invention gives proof in the present invention that the *in vivo* levels of low-level or unstable nucleic acid transcripts can only be measured/determined/quantified when starting from a pool of RNA prepared from stabilized whole blood, using a compound inhibiting extra-and/or intracellular RNA degradation and/or gene induction; whereby the isolation of the nucleic acids is performed using an automated device, whereby the reagent mix and the isolated nucleic acids, used for the RT-PCR reaction, are dispersed using an automated device, and whereby the determination of the transcript levels is performed in an automated setup. According to the present invention, only this approach allows to quantify *in vivo* leves of low-level or unstable RNA in a reproducible manner. The number of steps performed in said method is reduced to a minimum in order to avoid errors. An 'error' may be a pipetting-, a handling-, a procedural- and/or a calculation error or any error which can be made by a person skilled in the art. In this respect, the present invention suggests to perform the RT and the PCR reaction in one step. The method of the present invention will even be more accurate when combining more intermediate steps. For example, in the method of the present invention steps (a) and (b) can be combined.

According to the present invention, the dispersion of the nucleic acids (step (g)) may be performed after, before or simultaneously with the dispersion of the reagent mix needed for RT-PCR (step (f)).

According to the method of present invention, no OD measurements need to be performed, eliminating the errors made in the calculation of the nucleic acid concentration. Contrarily, using the PAXgene^{™} Blood RNA kit OD measurements need to be made. This illustrates again that the method according to present invention is a more reliable and accurate method compared to the latter system. This better accuracy of the present invention is illustrated by the reproducibility studies presented in Table 3.

According to the present invention, when dissolving the formed precipitate in step (d) of the method according to the present invention, the obtained suspension can be used in combination with an RNA extraction method and an analyzing method which are fully automated. It is only this combination which allows to optimize accuracy and reproducibility of the performed method and which allows to determine real *in vivo* RNA levels! of low-level or unstable RNA. As the brochure of the PAXgene^{™} Blood RNA System describes that the corresponding tubes can not be used in combination with other isolation methods, and no detailed information is available describing the different compositions of the kit, it is not obvious for a person skilled in the art to use parts of this PAXgene^{™} Blood RNA System and develop a new method therefrom.

There exist only few commercial systems which allow to isolate RNA fully automatically. Examples of such automated nucleic acid extractors are: the MagNA Pure LC Instrument (Roche Diagnostics), The AutoGenprep 960 (Autogen), the ABI PrismTM 6700 Automated Nucleic Acid Workstation (Applied Biosystems), WAVE® Nucleic Acid Analysis System with the optional WAVE® Fragment Collector FCW 200 (Transgenomic) and the BioRobot 8000 (Qiagen).

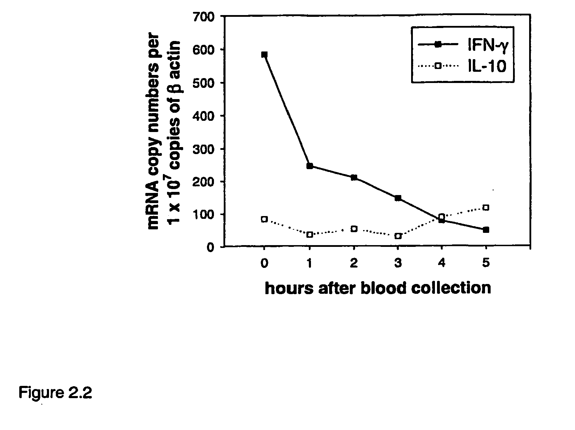

The present invention points towards the fact that for all these systems it is essential to start with material which is as fresh as possible or which is stabilized in order to allow the determination of real *in vivo* transcript levels of low-level or unstable RNA from whole blood. The problem for all these systems is that the blood is collected and brought to the laboratory in tubes that contain no or only a conventional additive, so that mRNA can still be rapidly degraded. Consequently, mRNA quantification using these methods will undoubtedly lead to the quantification of the transcripts present in the tube, but this quantification does not represent the transcript levels present in the cells/biological agent at the moment of sampling. Experimental evidence of this is provided in Figure 2.2 of example 1 of the present invention.

With the term 'quantification' is meant accurate and reproducible determination of RNA copy numbers; but it is trivial for a person skilled in the art that also qualitative or semiquantitative studies can be performed using RNA isolated via a method as described by the present invention.

The definition 'transcript' is not limited to messenger RNA (mRNA) but also relates to other types of RNA molecules known to exist by a person skilled in the art. According to the method of the present invention mRNA as well as total RNA can be extracted. This allows to get a correct estimation of the *in vivo* nuclear RNA, providing a powerful tool to evaluate gene transcription.

With 'whole blood' is meant blood such as it is collected by venous sampling, i.e. containing white and red cells, platelets, plasma and eventually infectious agents; the infectious agents may be viral, bacterial or parasitical. The clinical samples may be from human or animal origin. The sample analyzed can be both solid or liquid in nature. It is evident when solid materials are used, these are first dissolved in a suitable solution, which could be the RNAlater reagent sold by Qiagen. According to the invention, this solution is not always a real "buffer" with at least two well balanced components. It may be a strong hypotonic solution such as NaCl alone or an extraction solution such as with alcohol.

The term 'nucleic acid' refers to a single stranded or double stranded nucleic acid sequence, said nucleic acid may consist of ribonucleotides (RNA), RNA/DNA hybrids, or a combination thereof. A nucleic acid sequence according to the invention may also comprise any modified ribo nucleotide known in the art.

According to the present invention, the nucleic acid may be present extra- or intracellularly in the whole blood.

The 'separation' of the precipitate from the supernatant in step (c) of present method can be performed via centrifugation, filtration, absorption or other means known by a person skilled in the art. Said precipitate may include cells, cell/debris, nucleic acids or a combination thereof. The basis of the concept is to stop the nucleic-acid-containing-agent (or biological agent) from having contact with external sources/pulses/signals. This can be performed by fixing, lysing and/or disintegrating the nucleic-acid-containing-agent, or by any other means known by a person skilled in the art.

The buffer used in step (d) of the method of present invention may be a buffer to dissolve the precipitate obtained in step (c) of said method. This buffer may have additional effects such as lysis or further lysis of the nucleic-acid-containing-agent.

The 'automated device' used may be an automated pipetting device or another automated device known by a person skilled in the art suitable for carrying out the indicated actions.

With a 'reagent mix for RT-PCR' is meant all reagents needed for a simultaneous RT and PCR reaction (with the exception of the oligonucleotides when explicitly mentioned). According to the present invention, 'oligonucleotides' may comprise short stretches of nucleic acids as found in for example primers or probes. According to the present invention, the *in vivo* levels of the nucleic acids can be determined using real-time PCR or by any method allowing the determination of real *in vivo* RNA levels. According to the present invention, this method can be used in combination with micro-arrays or Rnase protection assays.

As pointed out before, storage of blood leads to incorrect mRNA levels. Indeed, in practice, the analysis of fresh sample is not feasible as the place of sampling and the place of RNA analysis is located differently. The method according to the present invention allows to transport blood samples without any effect on their *in vivo* transcript content. Transport of the blood sample can be performed after step (a) or step (b) in the method of the present invention.

Usually, red blood cells are preferentially eliminated before the nucleic acids are isolated. Red blood cells are rich in hemoglobin and their presence results in the production of highly viscous lysates. Therefore, removal of these allows to isolate nucleic acids in a more improved fashion. However, in the method of the present invention, this step is eliminated as an insoluble precipitate is immediately formed comprising the nucleic acids, separating these from all other components of the blood sample. This illustrates that, in addition to other advantages, the method of the present invention is a superior method in comparison with most prior art methods.

The present invention suggests to apply the PAXgene^{™} Blood RNA Tubes in the present method. These contain an additive that stabilizes cellular RNA and may eliminate *ex vivo* induction of the gene transcription. No detailed information is provided describing the content of this additive. The brochure refers to patent US 5,906,744 for this purpose. Nevertheless, the tube described in this patent allows a person skilled in the art to prepare nucleic acids from plasma and not from whole blood as performed in the present invention. In particular, the device of US 5,906,744 preferably comprises a plastic or glass tube, a means for inhibiting blood coagulation and a means for separating plasma from whole blood (US 5,906,744 column 2, I.42-43). Therefore, according to the present invention, the content as described in US 5,906,744 does not relate to the real content of the PAXgene^{™} Blood RNA Tube as it relates to a different use.

According to the present invention the content of these tubes may contain a quaternary amine surfactant. Therefore, according to the present invention, a quaternary amine surfactant may be used in step (a) of the method of the present invention. The use of a quaternary amine surfactant in order to stabilize nucleic acids in a biological sample has been previously described in US5,010,183. This patent provides a method for purifying DNA or RNA from a mixture of biological materials. Said method comprises the step of adding a cationic detergent to a mixture containing the RNA or DNA in an amount sufficient to dissolve cells, solubilize any contaminating proteins and lipids in the mixture, and form insoluble hydrophobic complex between the nucleic acid and the detergent. The complex which comprises the RNA or DNA with the detergent thus becomes separated from the solubilized contaminants. In a more recent patent, the same inventors stated that the use of the surfactant, as described in US 5,010,183, and other commercially available surfactants results in inefficient precipitation of RNA and incomplete lysis of blood cells. As there was a need for improved cationic surfactants for this purpose, the inventors of US 5,010,183 searched for a novel method for isolating RNA from a biological sample, including blood, involving the use of an aqueous, cationic surfactant solution comprising a selected quaternary amine (US 5,985,572). New aqueous quaternary amine surfactants, able to stabilize RNA from biological samples, are also described in WO94/18156 and WO02/00599. The synthesis of the different possible surfactants, that can be used in any methods of the present invention, can be performed according to the instructions as published in above cited or related patents. One example of a quaternary amine which can be used in the method of the present invention is tetradecyltrimethyl-ammonium oxalate. (US 5,985,572). Alternatively, said cationic detergent may be Catrimox-14^{™} (US5,010,183) as shown in the example 1 of the present invention. Further to the stabilization of said biological sample, said applications describe the isolation of the nucleic acids using conventional separation techniques such as column chromatography. Due to the obliged combination of the PAXgene^{™} Blood RNA Tube with the PAXgene^{™} Blood RNA kit (which also applies column chromatography) the supplier gives the impression that the compounds present in the PAXgene^{™} Blood RNA Tube may only be compatible with said chromatographic method.

According to the present invention, said compound of step (a) in any method of the present invention may be a compound inhibiting RNA degradation and/or gene induction as found in a PAXgene^{™} Blood RNA Tube.

The tube which can be used to collect the blood sample can be any tube. Therefore, in step (a) of the method according to the present invention, said tube may be an open or a closed blood collecting tube. Nevertheless, preferably a closed tube is used in order to prevent blood splatter, blood leakage and potential exposure to blood borne pathogens. A Hemogard^{™} closure may be used for this purpose (Becton Dickinson). Furthermore, blood is drained inside the PAXgene^{™} Blood RNA Tube by vacuum, so that the taken volume is always the same, allowing a "standardized sample volume".

According to the present invention, said buffer used in step (d) of the method of the present invention may be a guanidine-thiocyanate-containing buffer.

In the examples of the present invention the precipitate formed in the PAXgene^{™} Blood RNA Tubes is dissolved in the lysis buffer as provided by the MagNA Pure LC mRNA Isolation Kit I (Roche Diagnostics, Molecular Biochemicals). Therefore, it is suggested in the present invention that one of the possible buffers which may be used in the method of the present invention is a guanidine-thiocyanate-containing lysis buffer as provided by MagNA Pure LC mRNA Isolation Kit I (Roche Diagnostics, Molecular Biochemicals).

The MagNA Pure LC mRNA Isolation Kit I (Roche Diagnostics, Molecular Biochemicals) is especially designed for use on the MagNA Pure LC Instrument, to guarantee the Isolation of high quality and undegraded RNA from whole blood, white blood cells, and peripheral blood lymphocytes. According to its product description, obtained RNA is suitable for highly sensitive and quantitative LightCycler RT-PCR reactions, as well as for standard block cycler RT-PCR reactions, Northern blotting and other standard RNA applications. Nevertheless, the present invention proves that the use of this method 'as such' could not result in the determination of correct transcript levels. The present invention shows that there is a need to stabilize the RNA prior to the RNA isolation (see example 1). The present invention describes the unique combination of the use of RNA stabilizing compounds and an automated isolation/analysis procedure.

According to the present invention, once the precipitate of step (d) is dissolved in a lysis buffer such as the one provided by MagNA Pure LC mRNA Isolation Kit I, the method of the present invention may follow the procedure as described for the MagNA Pure LC mRNA Isolation Kit I. After the samples are lysed through the presence of a chaotropic salt in the lysis buffer, streptavidin-coated magnetic particles are added together with biotin-labeled oligo-dT, and the mRNA binds to the surface of the particles. This is followed by a DNase digestion step. mRNA is then separated from unbound substances using a magnet and several washing steps. Finally, the purified mRNAs are eluted. This isolation kit allows the automated isolation of pure mRNA as a "walk away" system. It allows to isolate mRNA of high quality and integrity suitable for all major downstream applications regarding gene expression analysis. Different protocols are offered, however before setting them on the MagNA pure LC Instrument. however, cells present in the blood samples are preferentially lysed manually before setting them mRNA isolation may then be postponed or directly further processed on the instrument.

The present invention proves in the present examples that the use of the MagNA Pure LC Instrument (Roche Diagnostics, Molecular Biochemicals) as automated device in step (e), step (f) and/or step (g) of the method according to the present invention leads to the production of a pool of RNA which can be used to determine exact/real *in vivo* levels of low-level or unstable transcripts. RNA-capturing beads such as magnetic beads, coated with otigo-dT via a streptavidin-biotin system or an equivalent system, may be applied in the method of the present invention in order to separate mRNA from the cellular debris.

Alternatively, according to the present invention other automated devices may be used such as the ABI PrismTM 6700 Automated Nucleic Acid Workstation (Applied Biosystems) or any other automated device that can be used for this purpose.

In the brochure of the MagNA pure LC mRNA Isolation Kit I (Cat No 3 004 015) no compositions of the buffers used in this kit are mentioned in detail. Therefore it is not obvious for a person skilled in the art to assume that the buffer as provided by this kit would allow to dissolve the pellet obtained by the method of the PAXgene^{™} Blood RNA Tubes. In addition, a person skilled in the art would not combine both methods based on the information provided by the PAXgene^{™} Blood RNA Tubes brochure stating that these tubes can only be combined with the corresponding PAXgene^{™} Blood RNA Kit (page 3, see limitations of the system; page 6, see ordering information).

As pointed out above, , red blood cells are preferentially lysed after step (a) in the method of the present invention. In the design of the MagNA Pure LC mRNA Isolation Kit I (Roche Diagnostics, Molecular Biochemicals) there is a possibility to lyse and eliminate red blood cells, before mRNA isolation from white blood cells.

Nevertheless, because of this step, samples cannot be treated fast enough to avoid mRNA degradation. The present inventors decided to use PAXgene^{™} Blood RNA Tube in conjunction with the MagNA Pure mRNA Isolation Kit on the MagNA Pure Instrument. Using the PAXgene^{™} Blood RNA Tubes provides a precipitate of nucleic acids that is not supposed to be soluble in the lysis buffer of the MagNA Pure mRNA Isolation Kit. Despite of this, the inventors found that it is actually possible. Following this observation, the inventors combined the use of the PAXgene^{™} Blood RNA Tubes with the use of an automated RNA isolation system. The inventors found surprisingly that this combination is possible and that this combination provides a powerful technique for the accurate mRNA quantification of low-level or unstable RVA from whole blood

The RNA isolated using the method according to the present invention is ready for use in a wide range of downstream applications, including for instance nucleic acid amplification technologies, such as RT-PCR and NASBA^{®}, Expression-array and expression-chip analysis, Quantitative RT-PCR, including TaqMan^{®} technology, cDNA synthesis, RNase and S1 nuclease protection, Northern, dot, and slot blot analysis and primer extension.

The present inventors showed in the example 1 and example 2 of the present invention that the use of a compound inhibiting RNA degradation and/or gene induction in conjunction with an automated RNA isolation and an automated analysis method such as real time PCR allows the determination of *in vivo* levels of transcripts. Nevertheless, according to present invention analysis methods other than real-time PCR may be applied as long as they are provided in an automated setup.

A main advantage of the method according to the present invention, is the fact that by using this method small sample volumes can be analyzed. This is of prime importance when only small volumes are available, for example when analyzing neonatal blood samples or in cases of high blood loss. According to the present concept RNA quantification may be performed using a blood sample as small as 100 µl. The analysis of RNA from a sample as small as 100 µl is not possible with the Qiagen kit (PAXgen^{™} Blood RNA System) which requires a larger volume of blood (2.5 ml following the kit handbook).
According to the present invention, the qualification may be performed using a whole blood sample g 20 to 200 µl.

The present application also describes a method for the quantification of *in vivo* levels of low level or unstable RNA from whole blood comprising the steps of:
(a) collecting a the whole blood in the PAXgene^{™} Blood RNA Tube,
(b) dissociating the surfactant/nucleic acid complex with a guanidine isothiocyanate buffer (this is not supposed to work based on the instruction manual of the PAXgene^{™} Blood RNA Tubes),
(c) extracting mRNA and/or total RNA using an reproducible automated device,
(d) dispersing/distributing a reagent mix for RT-PCR using an automated device,
(e) dispersing/distributing the nucleic acids isolated in step (c) within the dispersed reagent mix of step (d) using an automated device, and,
(f) quantifying the real in vivo levels of low-level or unstable RNA by real time PCR, whereby the RT and the PCR are preferably performed in one step, in order to avoid errors.

In this concept of the present invention, the automated device is any device that allows mRNA/RNA/DNA extraction from a guanidine isothiocyanate buffer, in a reproducible manner. The same or another may be used to accurately dispense the reagents and the samples in the reaction tube for the RT-PCR. An 'error' may be a pipetting-, a handling-, a procedural- and/or a calculation error or any error which can be made by a person skilled in the art.

The present invention also refers to a kit for the quantification of real *in vivo* levels of low-level or unstable RNA from whole blood comprising:
(a) a compound inhibiting RNA degradation and/or gene induction,
(b) an RNA precipitate dissolving buffer,
(c) reagents for automated RNA isolation,
(d) a reagent mix for a simultaneous RT and real-time PCR reaction or separate compounds thereof , allowing the automated dispersion of said mix, and,
(e) an instruction manual describing a method for an automated RNA isolation from whole blood a method for the automated dispersion of a reagent mix and the dispersion of the isolated nucleic acids for RT- real time PCR, and a method for automated RNA analysis, to quantify real in vivo levels of low-level or unstable RNA.

In the present examples the present inventors are applying the "Lightcycler mRNA hybridisation probes kit" from Roche Diagnostics, Molecular Biochemicals (cat # 3 018 954) to perform the RT-PCR reactions in one step. All reagents needed may be included in this kit, except the oligonucleotides (synthesized by Biosource). Nevertheless, real time PCR as described in the present invention can also be performed on other instruments such as the Applied Biosystems instruments.

According to the present invention, compound (a) of said kit may be a quaternary amine surfactant such as tetradecyltrimethyl-ammonium oxalate or may be a compound inhibiting RNA degradation and/or gene induction as found in a PAXgene^{™} Blood RNA Tube. The kit may additionally comprise a buffer such as a guanidine-thiocyanate-containing buffer which can be used in step (d) of the method according to the present invention.

The present application describes also a kit for isolating quantifiable *in vivo* levels of low-level or unstable RNA from whole blood comprising:
(a) a PAXgene^{™} Blood RNA Tube,
(b) a guanidine isothiocyanate buffer,
(c) reagents for automated RNA isolation,
(d) a reagent mix for a simultaneous RT and real-time PCR reaction or separate compounds thereof , allowing the automated dispersion of said mix, , and,
(e) an instruction manual describing a method for an automated RNA isolation from whole blood, a method for the automated dispersion of a reagent mix and the dispersion of the isolated nucleic acids for RT- real time PCR, and a method for automated RNA analysis, to quantify real in vivo levels a low-level orunstable RNA

According to the present application, a biological agent may be quantified which may be chosen from the group consisting of eukaryotic cells, prokaryotic cells, viruses and phages. According to the present application, the 'eukaryotic cell' may be any eukaryotic cell which is normally present or absent (eg. yeast, fungi, parasites or plant cells) in said sample; 'prokaryotic cells' may be bacteria; 'viruses' may be any RNA containing virus.

Nucleic acids which may be quantified using the methods of the present application may be chosen from the group consisting of nucleic acids coding for chemokines, cytokines, growth factors, cytotoxic markers, transcription factors, members of the TNF-related cytokine-receptor superfamily and their ligands, apoptosis markers, immunoglobulins, T-cell receptor, and any marker related to the activation or the inhibition of the immune system known or to be discovered.

According to the present application, said nucleic acids may code for a marker chosen from the group consisting of IL-1ra, IL-1β IL-2, IL-4, IL-5, IL-9, IL-10, IL-12p35, IL-12p40, IL-13, TNF-α, IFN-γ, IFN-α, TGF-β, and any interleukin or cytokine involved or not in the immune response. House keeping genes such β-actin or GAPDH (glyceraldehyde phosphate deshydrogenase) could be used as internal marker.

According to the present application said epitope specific CTLs-related or T Helper lymphocyte-related transcripts may be chosen from the group consisting of nucleic acids coding for cytokines, cytokine receptors, cytotoxines, Inflammatory or anti-inflammatory mediator, members of the TNF-related cytokine-receptor superfamily and their ligands, G-protein coupled receptors and their ligands, tyrosine kinase receptors and their ligands, transcription factors, and proteins involved in intra-cellular signaling pathways.

According to the present application, said nucleic acid may code for a marker chosen from the group consisting of granzyme, pertorines, prostaglandins, leukotrienes, immunoglobulin and immunoglobulin superfamily receptors, Fas and Fas-ligand, T cell receptor, chemokine and chemokine receptors, protein-tyrosine kinase C, protein-tyrosine kinase A, Signal Transducer and Activator of Transcription (STAT), NF-kB, T-bet, GATA-3, Oct-2.

The present application also describes a method/kit/procedure for the detection of *in vivo* immunological status for the diagnostic/prognostic of diseases affecting the immune system (cancer, auto-immune diseases, allergy, transplant rejection, GVHD, etc.)

According to the present application, the detection of cytokine mRNA (can be extended to chemokine, growth factors, cytotoxic markers, apoptosis markers, or any marker relate to the activation of the immune system known or to be discovered) with the described method in whole blood of patients suffering a disease that affects directly of indirectly their immune system with the aim to dress a diagnosis or prognosis.

According to the present application the 'immuno-related transcripts' may be transcripts coding for e.g. cytokine(s), chemokines(s), growth factors, cytotoxic markers, transcription factors, members of the TNF-related cytokine-receptor superfamily and their ligands, or any markers related to activation of the immune system known or to be discovered. According to the present application the immunomodulatory agent(s) may be present in case of a disease or in the presence of a transplant in said subject. The subject according to the present application may be of both human or animal origin.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can be used in the practice or testing of the present invention. In case of conflict, the present specification, including definitions, will control. The materials, methods, and examples are illustrative only and not intend to be limiting. Other features and advantages of the invention will be apparent from the following figures, detailed description, and from the claims.

### Brief description of the figures and tables

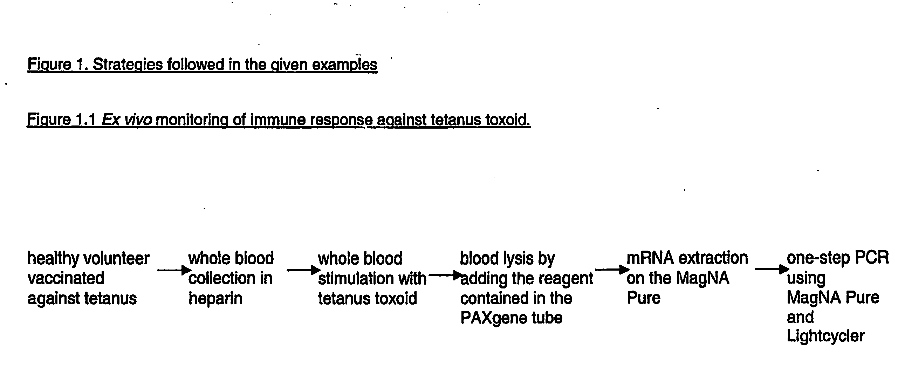

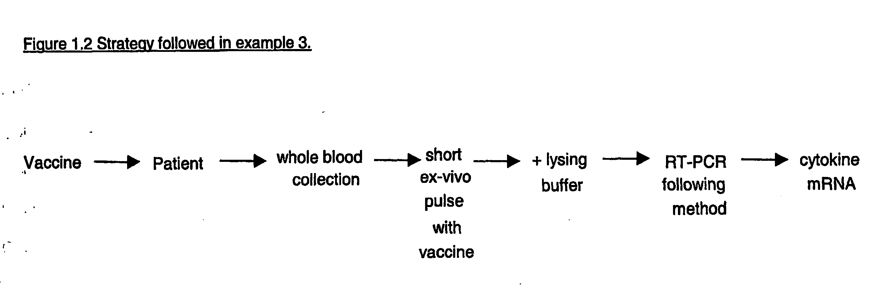

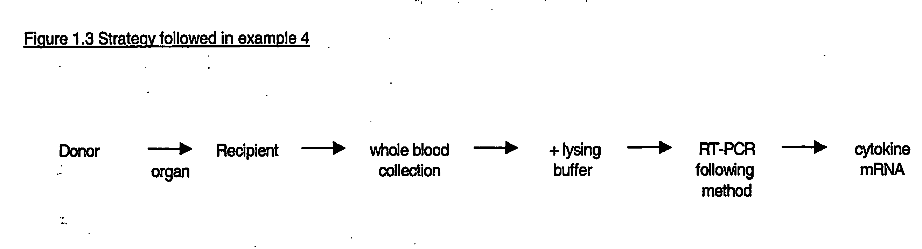

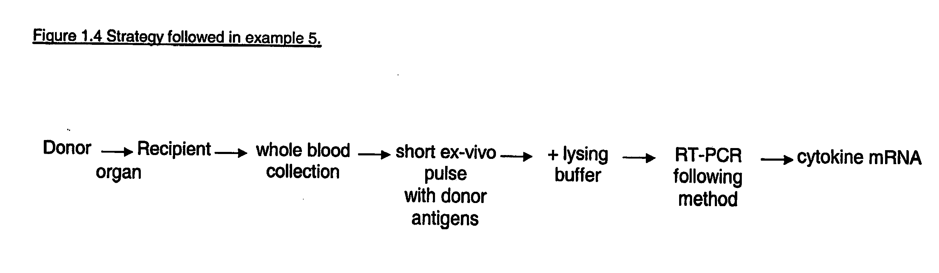

Figure 1. **Strategies followed in the given examples**
Figure 1.1 ***Ex vivo* monitoring of immune response against tetanus toxoid.**
Figure 1.2 **Strategy followed in example 3.**
Figure 1.3 **Strategy followed in example 4**
Figure 1.4 **Strategy followed in example 5.**

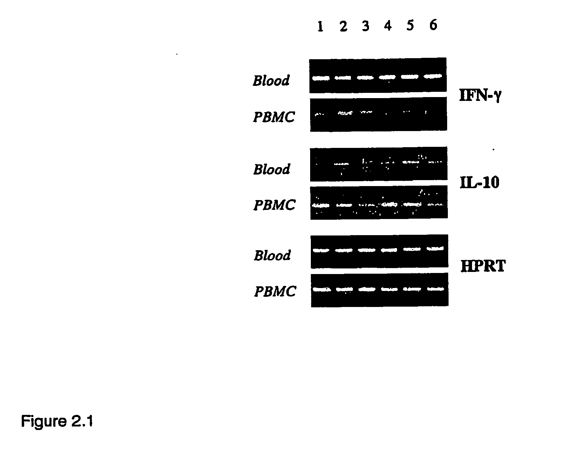

Figure 2.1: **RT-PCR for spontaneous production of IFN-.**γ **and IL-10 mRNAs in peripheral blood.** Total RNA was extracted from whole blood and from PBMC, as stated, from six different healthy volunteers (columns 1 to 6). Whole blood: 0.6 ml of whole blood were mixed with 6 ml of Catrimox-14™, within the minute that follows sample collection. The samples were then centrifuged at 12000 g for 5 min. The resulting nucleic acids pellet was carefully washed with water, and dissolved in 1 ml of Tripure™. RNA extraction was then carried out according to Tripure™ manufacturer's instructions. PBMC: cells were prepared following standard procedures from 15 ml of heparinized venous blood, and lysed in 1 ml of Tripure^{™} for RNA extraction. RT-PCR for IFN-γ, IL-10 and housekeeping gene HPRT were performed for all samples from 1 µg total RNA as described (Stordeur et al., (1995), Pradier et al., (1996)).
Figure 2.2: **Real time PCR for IEN-**γ **and IL-10 mRNA stability in whole blood.** A sample of citrated venous blood was collected from healthy donors. From this sample, a 100 µl aliquot was mixed with 900 µl of Catrimox-14™**,** within the minute that follows blood collection, and every hour after during five hours, the blood sample being simply kept at room temperature between each aliquot taking. The resulting nucleic acids pellet (see legend to Figure 2.1) was dissolved in 300 µl lysis buffer from the "MagNA Pure LC mRNA Isolation Kit I" (Roche Diagnostics, Molecular Biochemicals). mRNA was extracted using the MagNA Pure LC Instrument (Roche Diagnostics, Molecular Biochemicals) following manufacturers instructions (final elution volume: 100 µl). Reverse transcription and real time PCR were performed in one step, following the standard procedure described in the "Lightcycler - RNA Master Hybridisation Probes Kit" (Roche Diagnostics, Molecular Biochemicals), starting, from 5 µl of the mRNA preparation. Primers and probes sequences, and PCR conditions, are described in Stordeur et al, J Immunol Methods, 259 (1-2): 55-64, 2002) and Tables 1 and 2. Results are shown for one representative donor and expressed in mRNA copy numbers normalised against β actin.

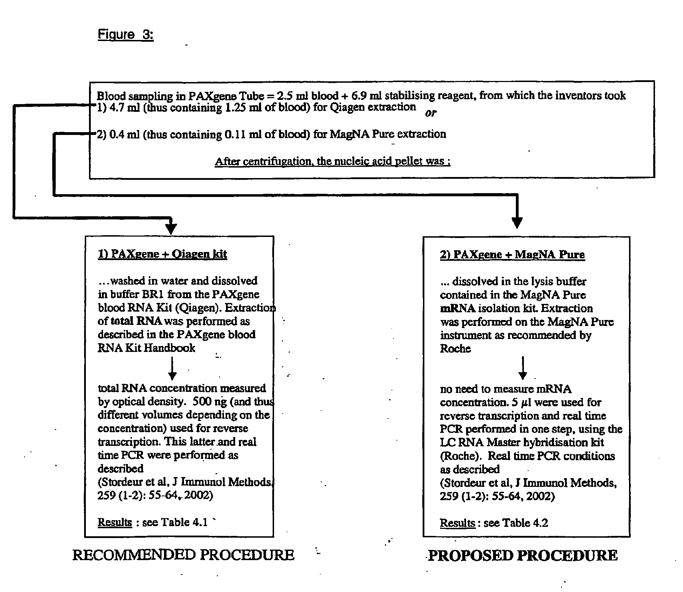

Figure 3: **Schematic comparison of the RNA extraction method from whole blood as suggested by PreAnalytiX compared to method as proposed by the present invention.**

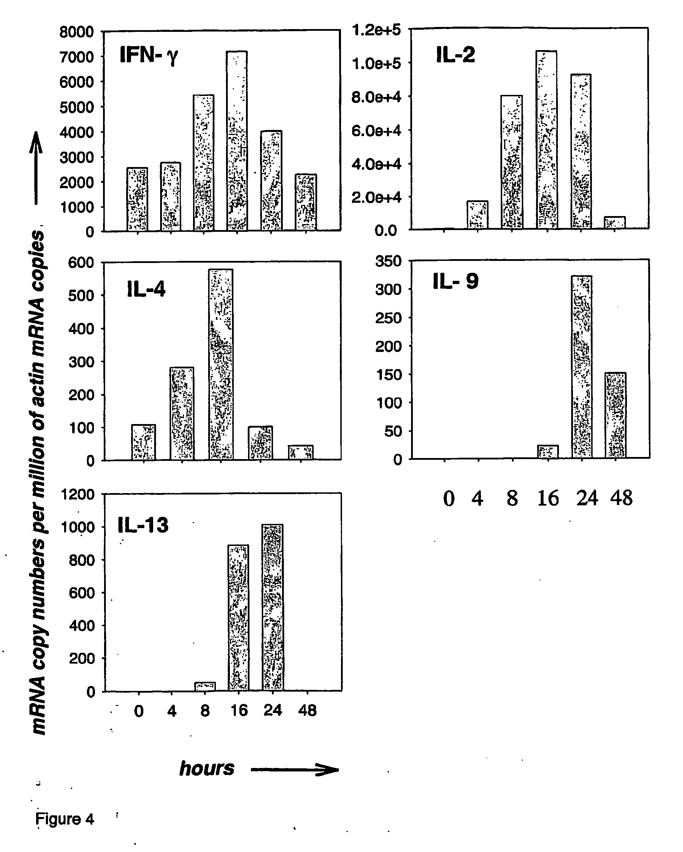

Figure 4. **Cytokine blood mRNA ex *vivo* induction by tetanus toxoid**. Tetanus toxoid (10 µg/ml, Aventis) was added to 500 µl whole blood collected from healthy volunteer vaccinated against tetanus seven years ago. After different time periods at 37°C in a 5% CO₂ atmosphere, 1.4 ml of the reagent contained in the PAXgene tube was added. 300 µl of the obtained lysate were used to isolate total mRNA on the MagNA Pure instrument, and RT-PCR was performed as described in the present invention.

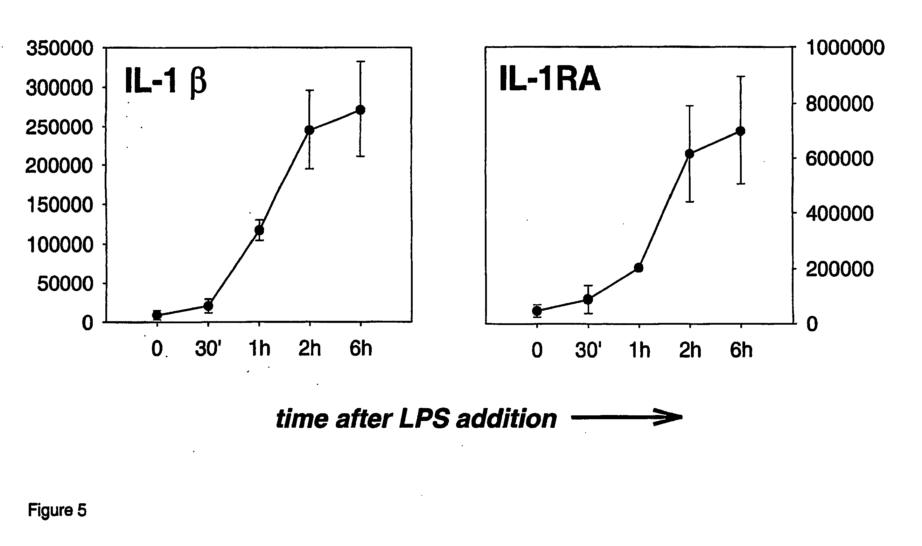

Figure 5. **IL-1**β **and IL-1 RA mRNA kinetics after whole blood stimulation with LPS.** 200 µl of heparinized blood were incubated with 10 ng/ml LPS for 0 (beginning of the culture), 0.5, 1, 2 and 6 hours. At the end of the culture, 500 µl of the PAXgene^{™} tube's reagent were added for total cell lysis and nucleic acid precipitation. Then RT and real time PCR for IL-1β, IL-1 RA and β-action mRNAs were performed in one step as described in the present invention. Results are expressed in mRNA copy numbers per million of β-actin mRNA copies. The mean and standard error on the mean of five independent experiments are shown.

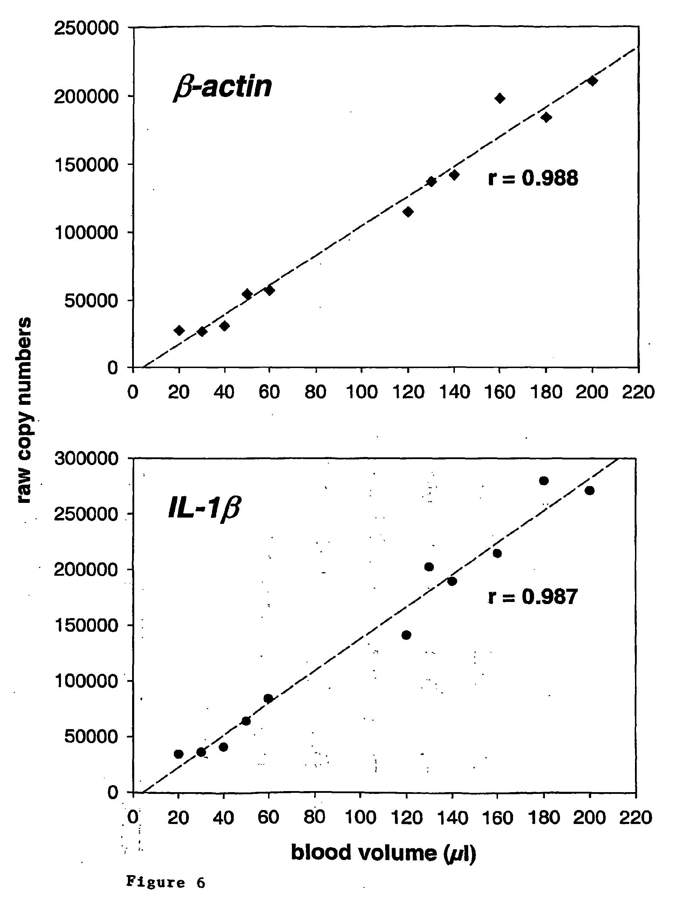

Figure 6. **Linear regression: mRNA copy numbers on starting blood volume.** Various whole blood volumes (ranging from 20 to 200 µl, X-axis) were cultured in the presence of 10 ng/ml LPS for six hours. At the end of the culture, RT and real time PCR for IL-1β and β-actin mRNAs were performed as described in the present invention. The Y-axis represents the raw copy numbers. The line is for linear regression. One experiment representative of six is shown.

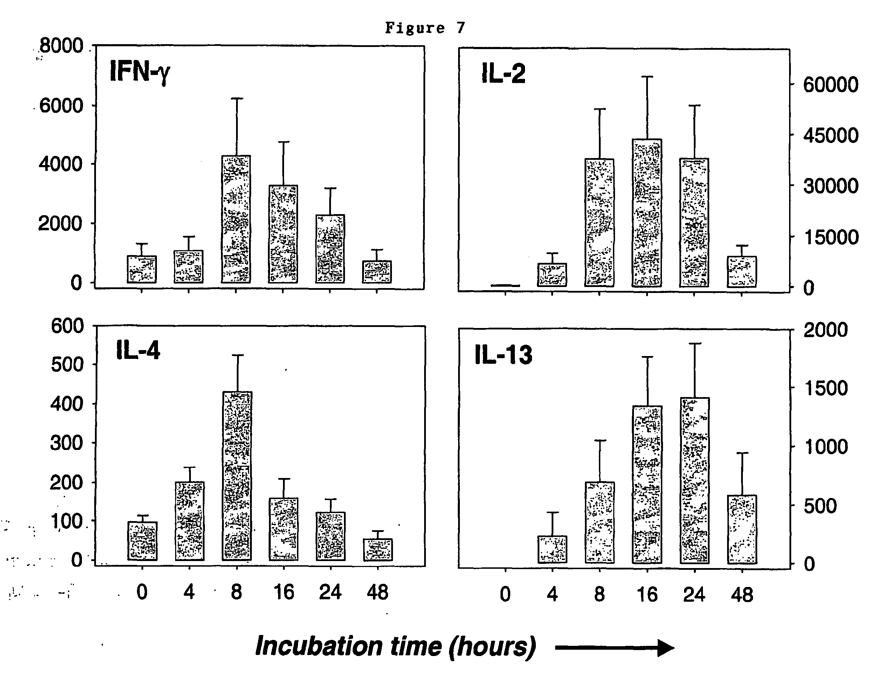

Figure 7. **mRNA cytokine kinetics after whole blood stimulation with tetanus toxoid.** Heparinized blood has been taken from five healthy volunteers who were vaccinated against tetanus at least five years ago. For each donor, 200 µl whole blood aliquots were incubated with 10 µg/ml tetanus toxoid for 0 (beginning of the culture), 4, 8, 16, 24 and 48 hours. At the end of the culture, 500 µl of the reagent contained in the PAXgene^{™} tube were added, and the different transcripts quantified using the methodology of the present invention. Results are expressed in mRNA copy numbers per million of β-actin mRNA copies. The mean and standard error on the mean of five independent experiments are shown.

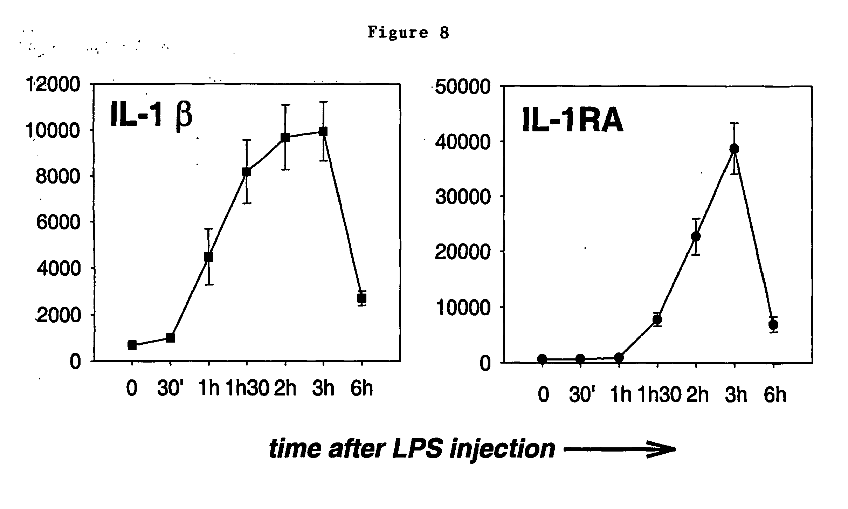

Figure 8. ***In vivo* modulation of blood cytokine mRNAs after intravenous injection of LPS.** Five healthy volunteers were injected with a single dose of 4 ng/kg LPS. Ten minutes before, and 0.5, 1, 1.5, 2, 3 and 6 hours after the LPS injection, a 2.5 ml sample of blood was taken in a PAXgene™ tube. Quantification of cytokine mRNAs was performed according to the method of the present invention. Results are expressed in mRNA copy numbers per million of β-actin mRNA copies. The mean and standard error on the mean for each time point are represented.

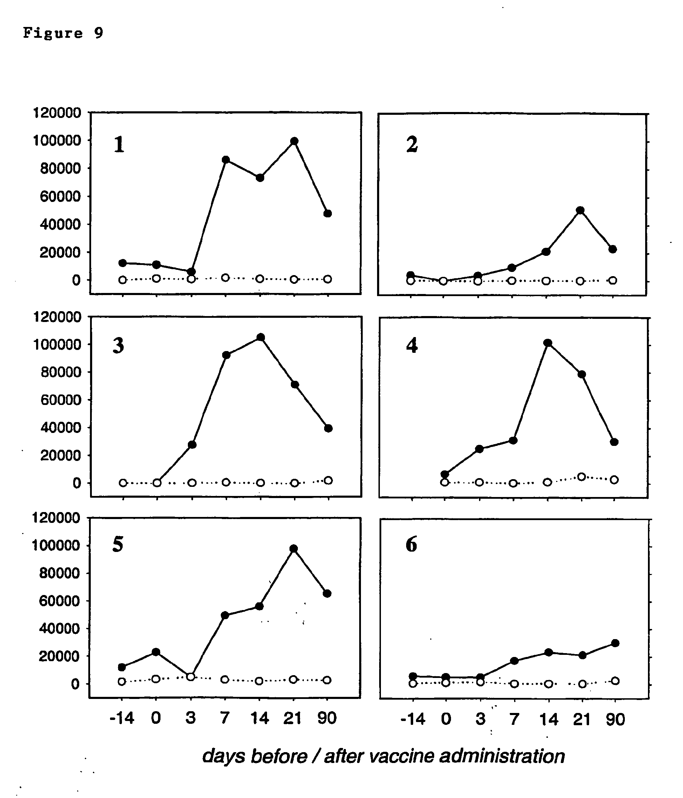

Figure 9. Follow-up of anti-tetanus vaccine response. Six healthy volunteers were selected to receive an anti-tetanus recall. IL-2 mRNA levels were quantified from whole blood cultured for 20 hours with (full circles) or without (open circles) 10 µg/ml tetanus toxoid, and performed at the moment of the recall (day 0), 14 days before, and 3, 7, 14, 21 and 90 days after (X-axis). Results are expressed in mRNA copy numbers per million of β-action mRNA copies (Y-axis). Each of the six panels (numbered 1 to 6) represents individual data from 6 different donors (one donor per panel).

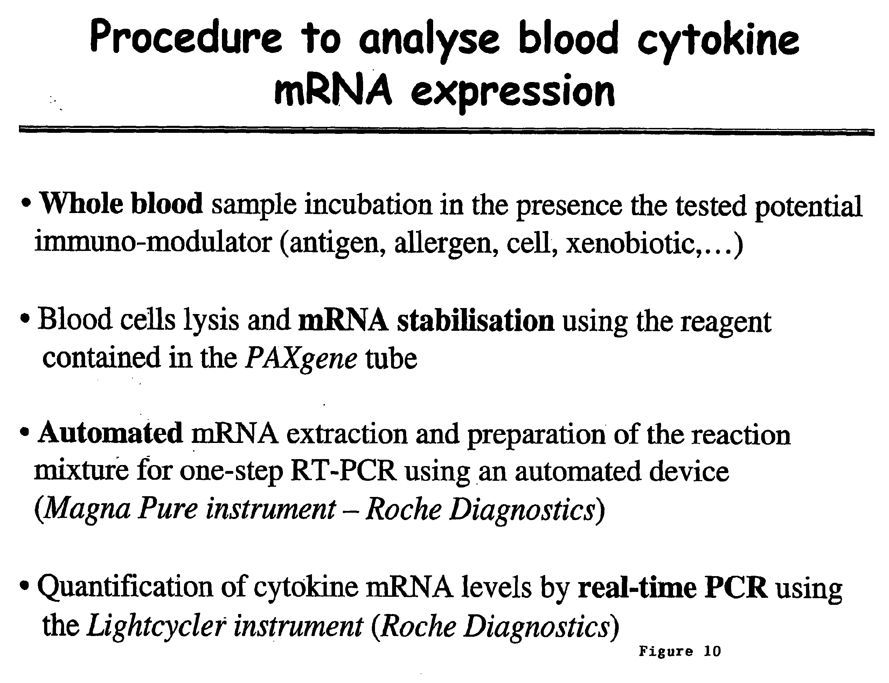

Figure 10. **Summary of the procedure followed in examples 7, 8, 9,10 and 11.**

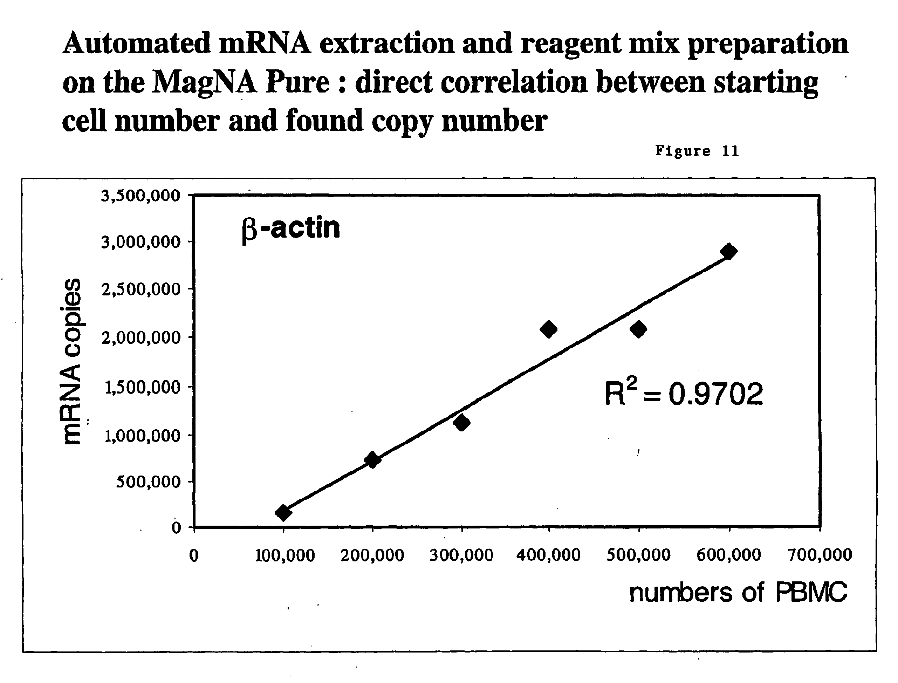

Figure 11. **Automated mRNA extraction and reagent mix preparation on the MagNA Pure.** direct correlation between amount of starting biological material and found copy number.

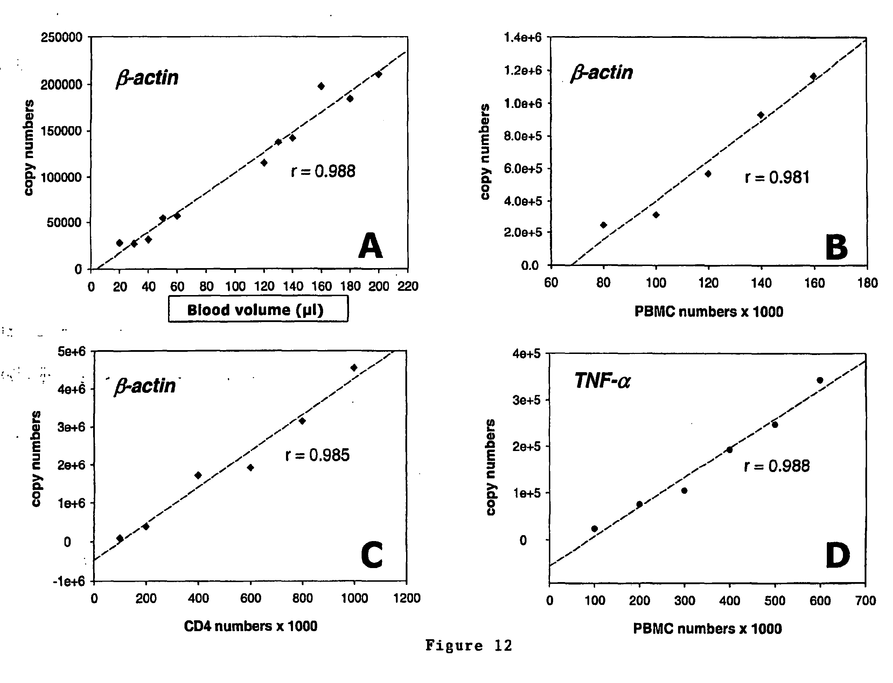

Figure **12****. Automated mRNA extraction and reagent mix preparation on the MagNA Pure.** The Y-axis represents the raw copy numbers. The line is for linear regression.

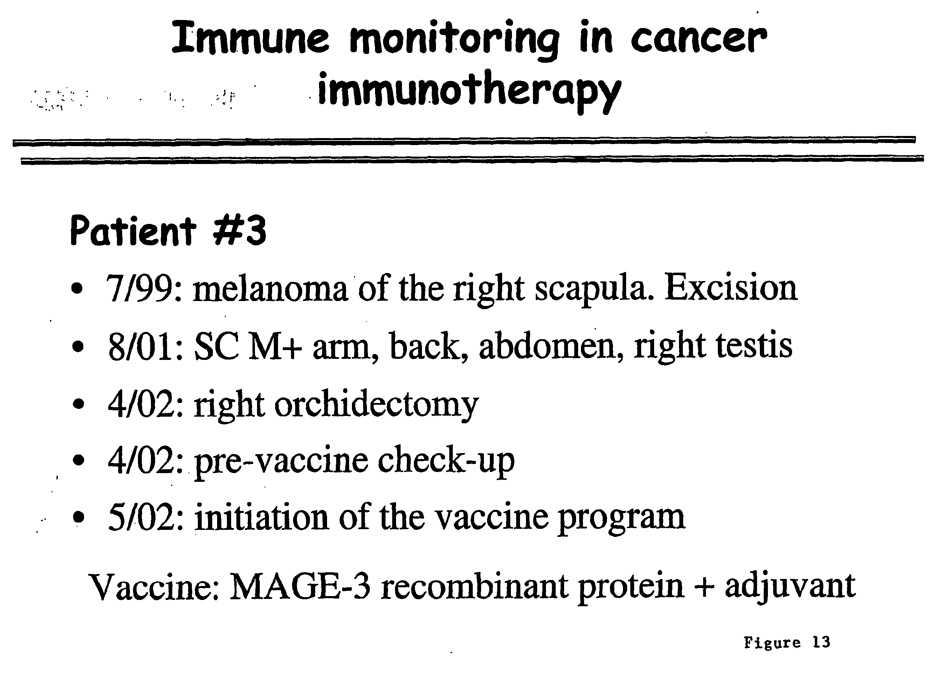

Figure **13****. Summarised case report of the patient enrolled for cancer immunotherapy.** The melanoma was diagnosed in July 1999. In Augusts 2001, multiple metastasis were evidenced; and directly after an orchydectomy in April 2002, the patient was enrolled for receiving a cancer vaccine. The vaccine consisted in several injections of the MAGE-3 purified protein (an antigen specifically expressed by melanoma cells) in combination with an adjuvant.

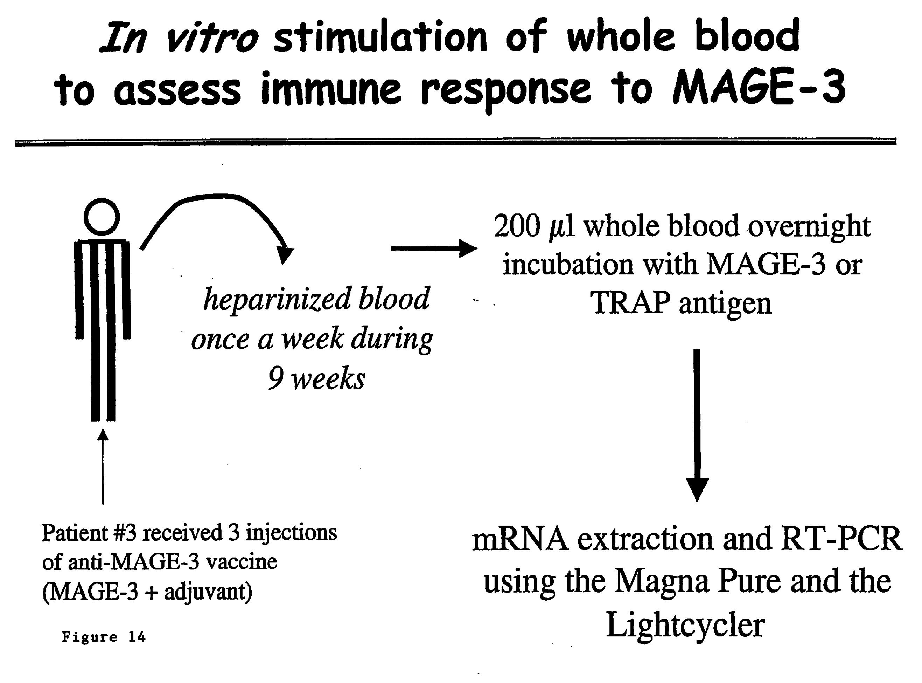

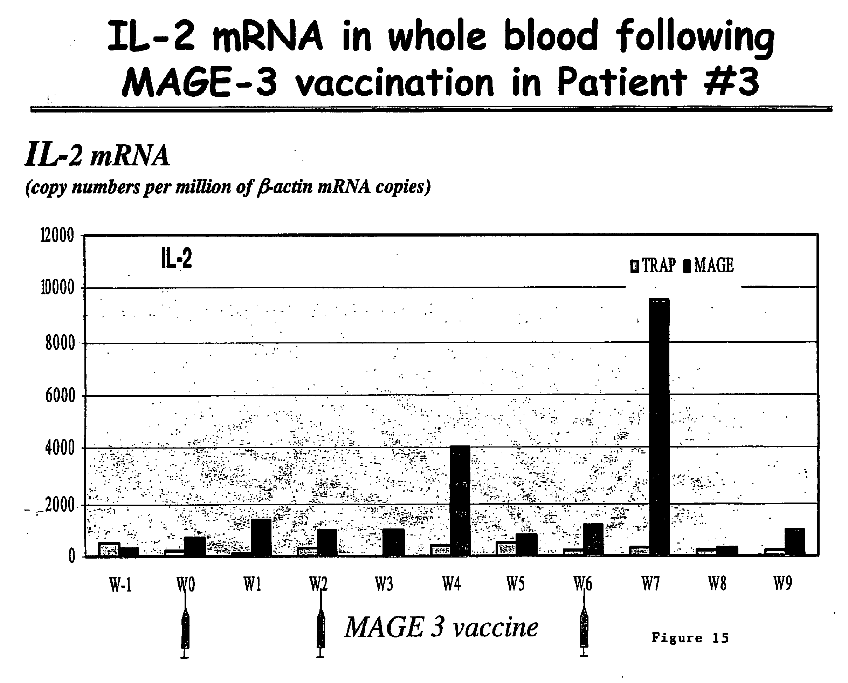

Figure 14. **Schematic representation of the vaccination protocol and the monitoring of immune response by real-time PCR.** The patient received 3 injections of the vaccine, while a blood sample was taken once a week during 9 weeks. A 200 µl aliquot of each patient's whole blood sample was incubated in the presence of 10 µg/ml MAGE-3 protein or 10 µg/ml TRAP (*plasmodium falciparum* antigen) as a negative control. At the end of the culture, the reagent contained in the PAXgene tube was added to allow IL-2 mRNA quantification as described in example 6. The results are presented in Figure 15.
Figure 15. **Higher IL-2 mRNA levels are observed in MAGE-3-stimulated whole blood after MAGE-3 vaccine boost.** The Y-axis represents the IL-2 mRNA copy numbers per million of β-actin mRNA copies, and the X-axis the weeks at which blood samples were taken. The vaccine injections were administrated at the weeks 0, 2 and 6. Dark red columns are for whole blood incubated in the presence of MAGE-3, and the blue columns for whole blood incubated in the presence of TRAP.

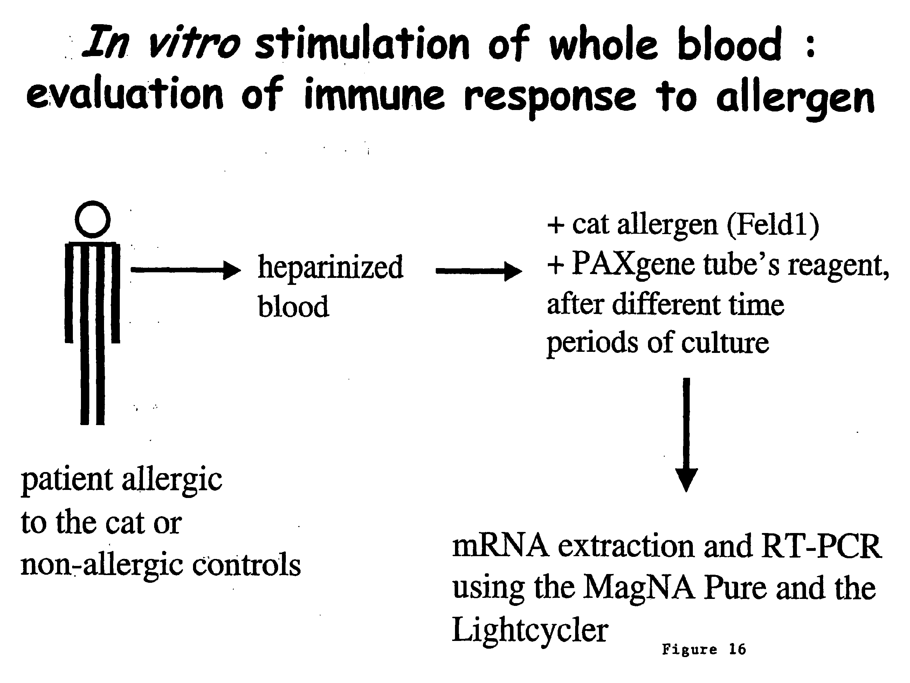

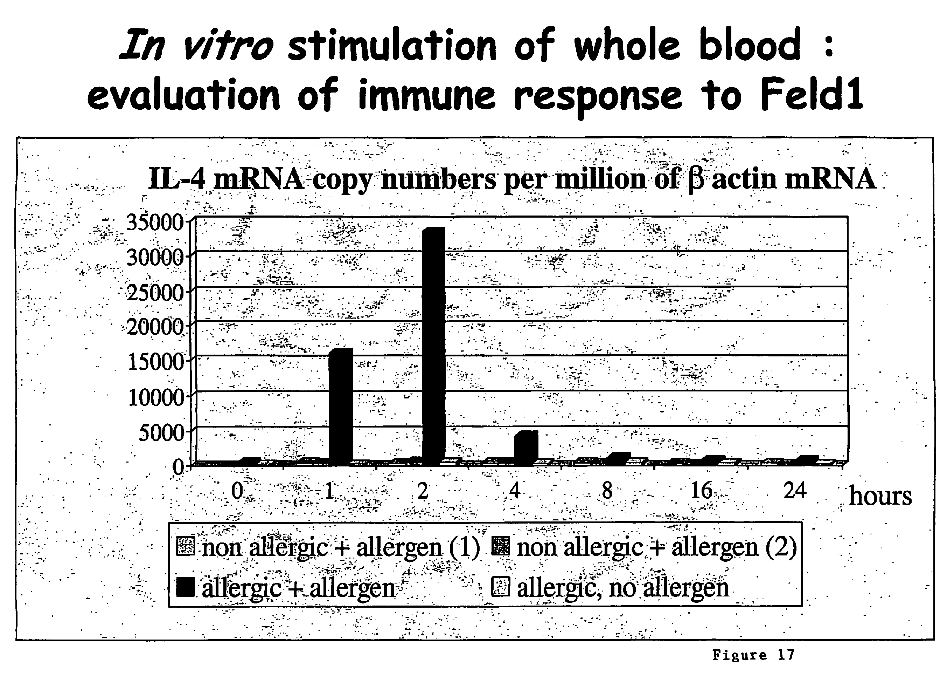

Figure 16. **Schematic representation of the experiment performed for IL-4 mRNA quantification after whole blood incubation with an allergen.** Blood samples were taken from a subject allergic to cat, and from two healthy subjects. Whole blood was then incubated in absence or in the presence of the cat allergen (namely Feld1), for different time periods of culture, at the end of which the reagent contained In the PAXgene tube was added to allow IL-4 mRNA quantification as described in example 6. The results are presented on Figure 17.
Figure 17. **Feld1 allergen significantly induces higher IL-4 mRNA levels In whole blood coming from the subject allergic to the cat compared to non allergic subjects.** The Y-axis represents the IL-4 mRNA copy numbers per million of β-actin mRNA copies, and the X-axis the different incubation times. Green columns represent IL-4 mRNA levels found in normal whole blood incubated with the allergen, IL-4 mRNA levels found in whole blood of the allergic subject being represented by the red columns (blood incubated in the presence of Feld1) and the yellow columns (blood incubated without Feld1).

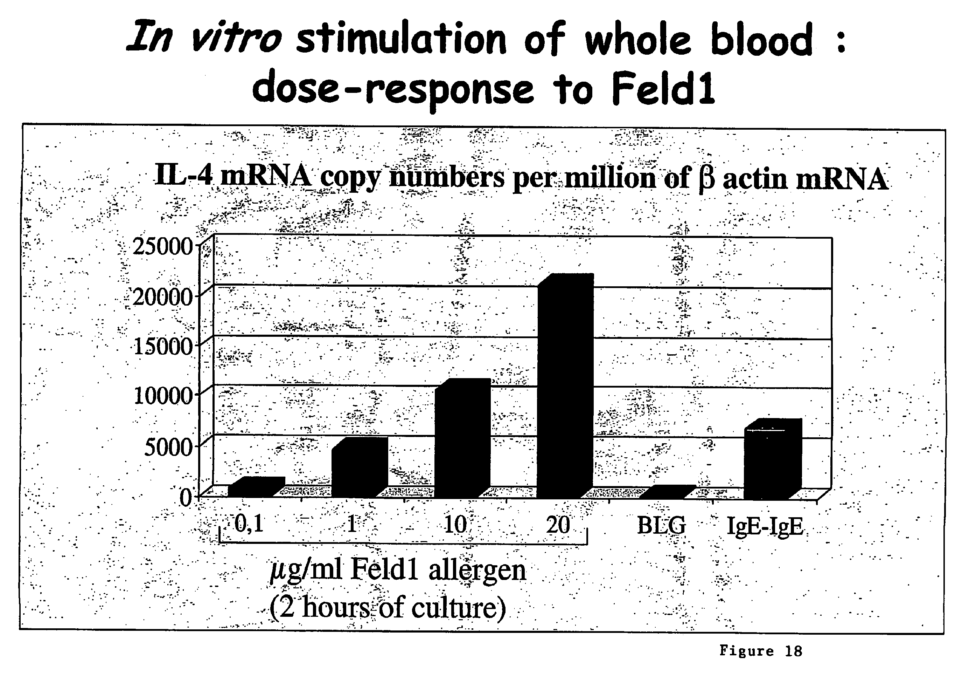

Figure 18. **The response to Feld1 In this whole blood system is specific and dose-related.** Whole blood from the allergic subject was incubated for two hours 1) in the presence of increasing concentrations of Feld1 (red columns); 2) in the presence of another allergen, β-lactoglobulin (BLG) at 10 µg/ml (blue column); 3) crossed-linked IgE (green column). The Y-axis represents the IL-4 mRNA copy numbers per million of β-actin mRNA copies.

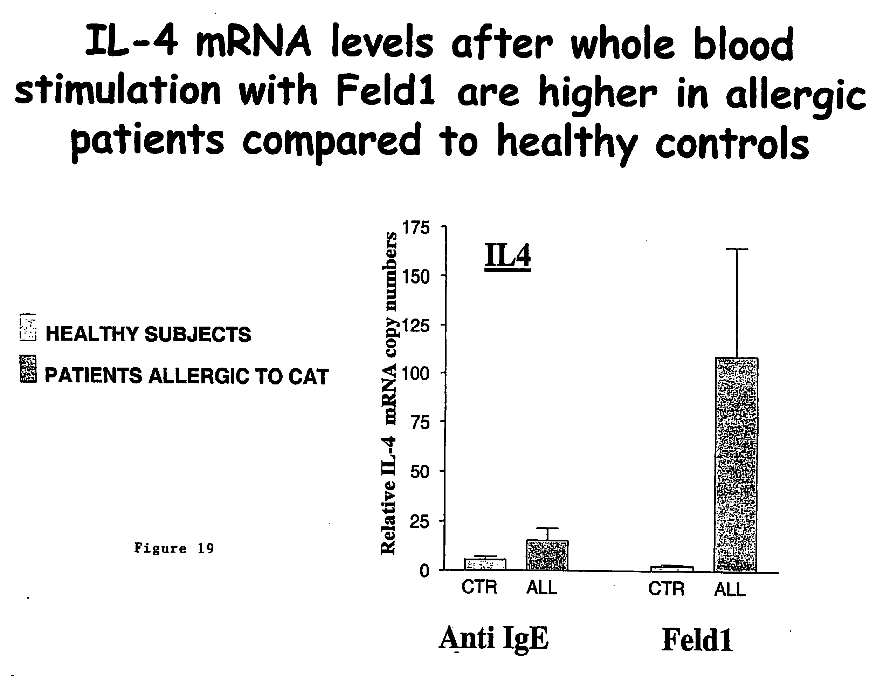

Figure 19. **IL-4 mRNA levels after whole blood stimulation with Feld1 are higher in patients allergic to the cat compared to healthy controls.** The experiment described on slides 9 to 11 was repeated on blood samples from 10 healthy subjects (CTR columns) and 10 patients allergic to the cat (ALL columns). Whole blood samples were incubated for two hours in the presence of 10 µg Feld1, or in the presence of crossed-linked IgE as positive controls. The mean and standard error on the mean are represented.

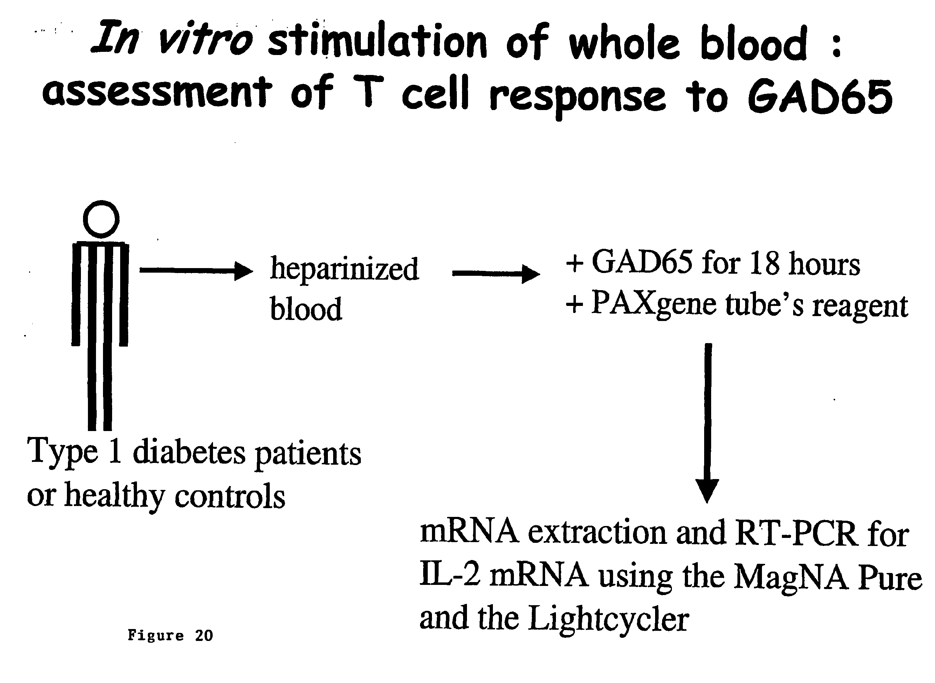

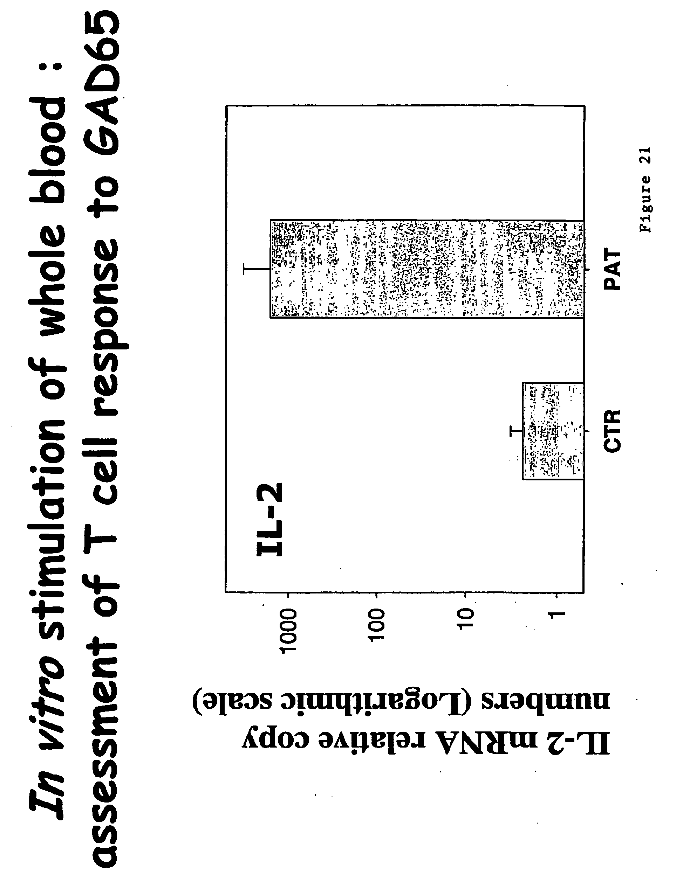

Figure 20. **Schematic representation of the experiment performed for IL-2 mRNA quantification after whole blood incubation with purified GAD65 protein.** Blood samples were taken from six type 1 diabetes patients, and from five healthy subjects. Whole blood was then incubated without or with 10 µg/ml GAD65 for 18 hours, the culture being then stopped by adding the reagent contained in the PAXgene tube. IL-2 mRNA levels were then quantified as described in example 6. The results are presented in Figure 21.
Figure **21****. Whole blood from type 1 diabetes patients shows higher IL-2 mRNA levels after GAD65 stimulation compared to healthy subjects.** Results are expressed in IL-2 mRNA copy numbers calculated relatively to the copy numbers found in whole blood cultured without GAD65, after correction against β-actin. A logarithmic scale is used. The mean and standard error on the mean are represented. Healthy donors: CTR column; autoimmune diabetes patients: PAT column.

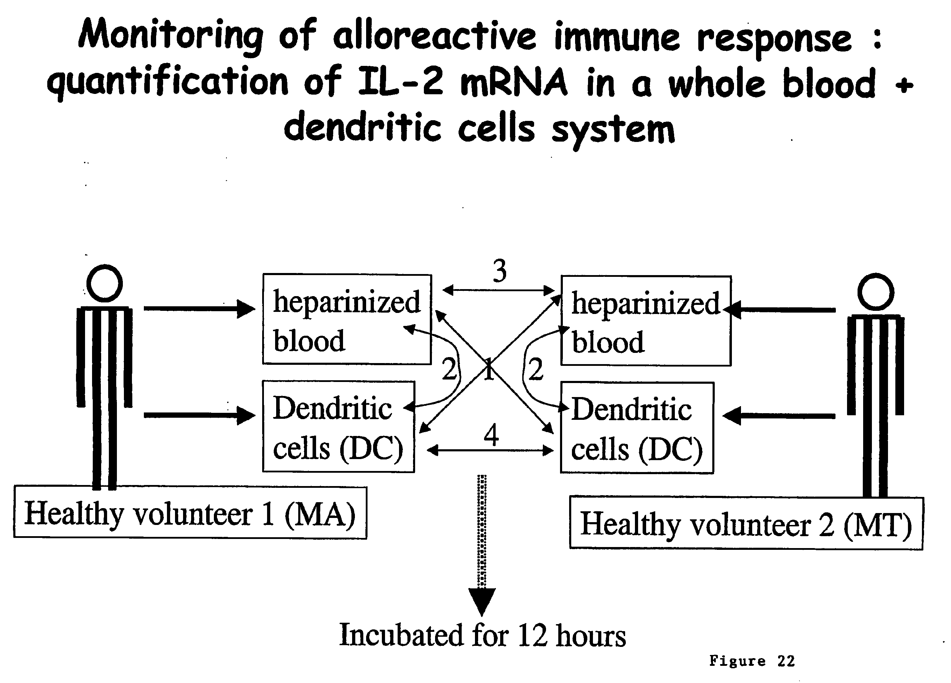

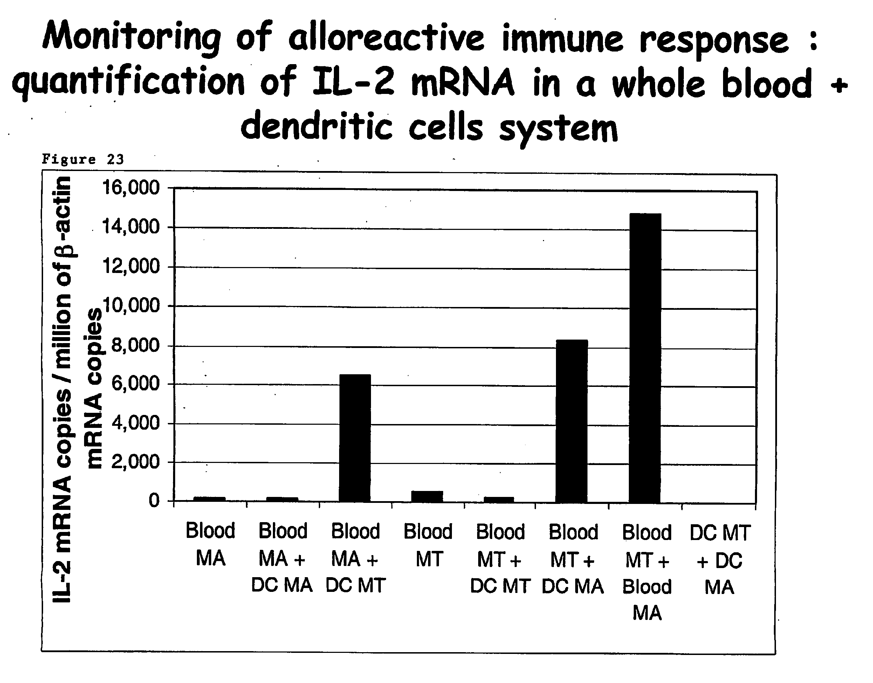

Figure 22. **Schematic representation of the experiment performed for IL-2 mRNA quantification after whole blood incubation with unrelated dendritic cells (DC) to assess alloreactive T cell response.** Dendritic cells from two unrelated healthy volunteers (MT and MA) were generated *in vitro* in the presence of IL-4 and GM-CSF. A whole blood sample from each donor was cultured in the presence of the dendritic cell population of the other donor (1) or in the presence of their own dendritic cells (2). Whole blood samples from both donors were mixed (3), as well as both dendritic cell preparations (4). After 12 hours incubation, the cultures were stopped by adding the reagent contained in the PAXgene tube. IL-2 mRNA levels were then quantified as described in example 6. The results are shown on Figure 23.
Figure 23. **Assessment of alloreactive T cell response by IL-2 mRNA quantification in whole blood.** IL-2 mRNA copy numbers per million of β-actin mRNA copies are shown. The conditions are, from left to right whole blood from donor MA alone, whole blood from donor MA + DC from donor MA, whole blood from donor MA + DC from donor MT, whole blood from donor MT alone, whole blood from donor MT + DC from donor MT, whole blood from donor MT + DC from donor MA, whole blood from donor MT + whole blood from donor MA, DC from donor MT + DC from donor MA.
Table 1: **Oligonucleotides for real time PCR used in** Stordeur et al, J Immunol Methods, 259 (1-2): 55-64, 2002**.**
Table 2: **Oligonucleotides for standard preparation used in** Stordeur et al, J Immunol Methods, 259 (1-2): 55-64, 2002**.**
Table 3: **Comparison of Qiagen and MagNA Pure LC mRNA extraction methods.**
Table 4: **Oligonucleotides for (real time) PCR of IL-2 and IL-4 target mRNA.**
Table 5. **IL-2 mRNA levels in response to tetanus toxoid: comparison of cord blood to adult whole blood.** 200 µl of heparinized cord blood were incubated for 20 hours with or without 10 µg/ml tetanus toxoid. Quantification of IL-2 mRNA levels was then performed as described. Results are compared to those obtained with adult whole blood taken just before vaccine recall (day 0, see legend to Figure 9). The mean ± SD of IL-2 mRNA copy numbers per million of β-actin copies are represented (n =3 for cord blood and 6 for adult blood).

### Modes for carrying out the invention:

### Example 1: Analysis of Spontaneous Cytokine mRNA Production in Peripheral Blood

The quantification of the cytokine mRNAs synthesized by peripheral blood cells should make it possible to estimate a "peripheral immune statute". However, an accurate quantification can only be performed from a fresh whole blood sample in which mRNA is protected against nuclease digestion, and where gene transcription is inhibited. As discussed in this note, this has been made possible by the use of surfactant reagents such as tetradecyltrimethylammonium oxalate. RT-PCR for the quantification of IL-10 and IFN-γ mRNAs spontaneously produced in peripheral blood was performed. The results showed pronounced higher IFN-γ transcript levels in whole blood compared to peripheral blood mononuclear cells (PBMC) from the same individuals, while no significant difference was observed for IL-10 mRNA. The higher amounts of IFN-γ mRNA observed in blood can be attributed at least to mRNA degradation. Using a real time PCR technique, it could indeed be demonstrated that blood IFN-γ mRNA is rapidly degraded *in vitro,* the *t* ½ being worth approximately one hour at room temperature.

Härtel et al. recently analysed the influence of cell purification procedure on spontaneous cytokine mRNA production in peripheral blood (Hartel et al., 2001). They showed that freshly isolated peripheral blood mononuclear cells (PBMC) expressed higher levels of IL-2, IL-4 and TNF-α mRNA than freshly collected whole blood from the same individual, while no difference in IFN-γ mRNA level was observed. A comparison for IFN-γ in six different individuals was performed, and different results were found. A strong expression of IFN-γ mRNA in whole blood of all donors was observed, which is clearly decreased in PBMC (Figure 2.1). This difference between the results obtained and those of Härtel et al, despite the fact that these latter used a quantitative real time PCR technique, could be related to the procedure used to isolate total RNA from whole blood. Härtel et al. used heparinized blood that was hemolyzed within two hours by isotonic ammonium chloride treatment. In the present method tetradecyltrimethylammonium oxalate was used, a cationic surfactant reagent called Catrimox-14™ (Qiagen, Westburg, Leusden, The Netherlands) that is directly mixed with the blood, avoiding the use of anticoagulants (Dahle and Macfarlane, (1993); Schmidt et al., (1995)). Moreover, this reagent induces nucleic acids precipitation and nuclease inhibition, in the minute that follows sample collection. This provides a total RNA preparation that is probably the nearest of *in vivo* mRNA status. This is especially important for cytokine mRNA, which are made sensitive to endogenous nucleases by their AU-rich sequences located in their 3' untranslated region. Using a real time PCR technique, it was indeed observed that peripheral blood IFN-γ mRNA is spontaneously and rapidly degraded, the levels being decreased by roughly 50 % already one hour after blood collection. However, this phenomenon is not necessary true for all the cytokines, as it was found that IL-10 mRNA level is stable for at least the five hours that follow blood sampling (Figure 2.2). Moreover, no significant differences in whole blood IL-10 mRNA levels were found, compared to those of PBMC (Figure 2.1).

The nucleic acids pellet obtained after Catrimox-14™ lysis (see legend to Figure 2.1) can be dissolved in the guanidium/thiocyanate solution described by Chomczynski and Sacchi (1987), as well as in its commercially available version, such as Tripure™ Roche Diagnostics, Molecular Biochemicals, Brussels, Belgium), making the use of this surfactant particularly easy. This means that, except for the first step with Catrimox-14™, the RNA isolation procedure is the same for whole blood and cells. Alternatively, PAXgene™ Blood RNA Tubes (Qiagen, Westburg, Leusden, The Netherlands) could be used in the place of Catrimox-14™. In this case, the resulting pellet can be dissolved in the lysis buffer of the "MagNA Pure LC mRNA Isolation Kit I", as described for Catrimox-14™ in legend to Figure 2.2. The characterisation of spontaneous IL-10 mRNA production by human mononuclear blood cells (Stordeur et al., (1995)), and the monitoring of *in vivo* tissue factor mRNA induction by OKT3 monoclonal antibody (Pradier et al., (1996)), represent two examples where Catrimox-14 was successfully used. A strong IL-2 mRNA induction was also observed after addition of ionophore A23187 + phorbol myristate acetate to whole blood (not shown), suggesting its use for *in vitro* studies on whole blood.

The observations made in the present example stress the importance to perform RT-PCR from whole blood lysed as fast as possible, in order to accurately quantify peripheral blood cytokine mRNA. For this purpose, the use of reagents such as Catrimox-14 or the additive contained in the PAXgene™ Blood RNA Tubes, together with real time RT-PCR, probably represents to-date the best procedure. By doing so, the study of the natural status of peripheral blood cells would be possible without the use of *in vitro* strong stimuli such as ionomycin or phytohaemagglutinin.

### Example 2: Comparison between the PAXgene^{™} Blood RNA System and proposed method according to the present invention.

With the 'PAXgene^{™} Blood RNA System' is meant the combination of the PAXgene^{™} Blood RNA Tube' with the 'PAXgene^{™} Blood RNA Kit'. With the 'Qiagen Method', it is meant 'PAXgene^{™} Blood RNA Kit'.

Based on the experimental evidence described in Stordeur et al, J Immunol Methods, 259 (1-2): 55-64, 2002, the present invention proposes a new procedure to isolate mRNA from whole blood which allows to determine *in vivo* transcript levels using an easy and reproducible method. The PAXgene^{™} blood RNA System and the method according to present invention are schematically compared in Figure 3.

### Material and methods:

All experiments were performed from peripheral venous blood directly collected in PAXgene^{™} Blood RNA Tubes as recommended by the PAXgene^{™} Blood RNA System (Qiagen) (*i*.*e*. 2.5 ml of blood were vacuum collected within the tube that contains 6.9 ml of an unknown reagent). After lysis completion, the content of the tube was transferred in two other tubes : 4.7 ml were used for PAXgene blood RNA kit, and 0.4 ml for MagNA Pure extraction. The remaining of the lysate was discarded. These two tubes were centrifuged at 2,000 g for 10 min and the supernatant discarded. The nucleic acid pellet was then:
***a) PAXgene^{™} Blood RNA Tube + PAXgene^{™} Blood RNA Kit-*** ... washed in water before being dissolved in BR1 buffer for total RNA extraction, as recommended in the corresponding instruction manual. The procedure of the PAXgene^{™} Blood RNA System is as follows: Blood samples (2.5 ml) are collected in PAXgene Blood RNA Tubes, and may be stored or transported at room temperature if desired. RNA isolation begins with a centrifugation step to pellet nucleic acids in the PAXgene Blood RNA Tube. The pellet is washed, and Proteinase K is added to bring about protein digestion. Alcohol is added to adjust binding conditions, and the sample is applied to a spin column as provided by the PAXgene^{™} Blood RNA Kit. During a brief centrifugation, RNA is selectively bound to the silica-gel membrane as provided by the PAXgene^{™} Blood RNA Kit as contaminants pass through. Following washing steps, RNA is eluted in an optimized buffer. Reverse transcription and real time PCR for IFN-γ and β-actin mRNAs were conducted as described by Stordeur et al. ("Cytokine mRNA Quantification by Real Time PCR" J Immunol Methods, 259 (1-2): 55-64, 2002).
b) ***PAXaene*^{™} *Blood RNA Tube + + MagNA Pure LC mRNA Isolation Kit I -* ...** dissolved in 300 µl lysis buffer from the MagNA Pure mRNA Isolation Kit. Extraction and purification of mRNA in a final elution volume of 100 µl were then performed on the MagNA Pure LC Instrument following the instructions from Roche Diagnostics, Molecular Biochemicals.
   Reverse transcription and real time PCR were conducted in one step, following the standard procedure described in the "Lightcycler - RNA Master Hybridisation Probes Kit" (Roche Diagnostics, Molecular Biochemicals), starting from 5 µl of the mRNA preparation.

### Results:

A comparison of the extraction method recommended by Qiagen in combination with the PAXgene^{™} Blood RNA Tubes (PAXgene^{™} Blood RNA System), with the MagNA Pure LC Instrument extraction method also in combination with the PAXgene^{™} Blood RNA Tubes was performed. In both methods the use of the PAXgene^{™} blood RNA Tubes allows to stabilize RNA from blood cells. The results are listed in Table 3.1 and 3.2. The results of this experiment show a better reproducibility for the MagNA Pure LC Technique (coefficients of variation for IFN-γ mRNA copy numbers corrected against β-actin are 26 versus 16 % for Qiagen versus MagNA Pure LC, respectively).

It is interesting to note that MagNA Pure extraction was performed from a starting blood volume lower than that used with the Qiagen method (0.11 ml for MagNA Pure versus 1.25 ml for Qiagen). If the Qiagen method had been performed with such small volume, it would be impossible to measure the RNA concentration, even to perform the reverse transcription. This stresses another advantage of the technique described in the present invention : the possibility to quantify mRNA in a very small volume of blood (about 100 µl).

### Conclusion:

Example 2 illustrates the possibility to use the PAXgene^{™} Blood RNA Tubes in combination with the MagNA Pure LC mRNA Isolation Kit I, or more precisely, the possibility to dissolve the precipitate from the PAXgene^{™} Blood RNA Tube in the lysis buffer contained in that kit, this lysis buffer necessarily having to be used with the other components of the kit.

In this example it is proven that in contrast to other combinations, only the combination as described in the present invention, leads to correct/real *in vivo* transcript quantification.

### Example 3: Ex vivo monitoring of immune response against tetanus toxoid.

In example 3, blood is stimulated *ex vivo* with an antigen (i.e. tetanus toxoid) against which the blood donor is supposed to be immunised (because vaccinated seven years ago). RT-PCR is performed according to the method (Figure 1.1). Cytokine mRNA is measured as a read out of the ability of the volunteer's immune system to react against the antigen. The IL-2, IL-4, IL-13 and IFN-γ mRNAs are preferentially analysed, but all potentially reactive proteins can be analysed via the quantification of their corresponding mRNA. Results of example 3 is shown in Figure 4. Generally the strategy followed in this example can be schematically represented as shown in Figure 1.2.

### Example of possible application: Cancer immunotherapy

Since some years, basic strategies on cancer immunotherapy evolved in the way of the vaccination. In fact, the progresses in genetic and in immunology have allowed identifying a number growing tumor antigens that are expressed to the surface of tumor cells. These antigens are presented to the surface of tumor cells under the form of peptides associated to the major histocompatibility complex (HLA). Example of antigens that might be considered as tumor antigens are described by Fong and Engleman (Annu. Rev. Immunol. 2000. 18:245-273). The principle of the anti-cancer vaccination consists to present these antigens to the system immune of the patient following the most immunogenic way immunogenic. That goes from the injection of the antigen or corresponding peptides in the presence of additives to the presentation of the peptide on autologous antigen presenting cells (dendritic cells, for example). Although the ultimate goal of vaccination anti-cancer vaccination remains the regression of the tumor, the determination of the efficiency of anti-cancer vaccination remains difficult especially in the case of patients in advanced phase of the disease that can profit only from a limited window of treatment. It is the reason why the anti-cancer vaccination could especially be interesting as adjuvant therapy or in the framework of the prevention. It is therefore extremely important to develop sensitive and precise monitoring techniques to evaluate the immunological effects of the experimental anti-cancer vaccination in order to specify the method of administration of these vaccines and discover the implied biological mechanisms that will be able to help better to define the futures therapeutic protocols. The difficulty to measure the immunological efficiency of these vaccines resides essentially in the absence of assays sufficiently sensitive to detect a cellular immune response *in vivo.* Until now, the used techniques implied the intensive *in vitro* culture of the PBMC of patients on of long periods times in the presence of antigen and of co-stimulating susceptible to induce a modification of the original functional characteristics of lymphocytes. Thus, the analyses of the anergic states or tolerant states of the lymphocyte precursors directed against the tumor antigens is extremely difficult being given the reversible nature of their functional state after their extended *in-vitro* incubation in the presence of antigen. On the other side, techniques based on tetramers of MHC-peptides complexes that are used for the detection of low frequencies of epitope-specific-CTL precursors lack usually sensitiveness for the detection of tumor-specific lymphocytes. In addition these techniques do not give any information on the functional reactivity of these lymphocytes

Only techniques that are sensitive enough to be able to detect an original functional reactivity of the lymphocytes to a given antigen, for example after a very short stimulation *in vitro* with antigen will allow a real evaluation of the efficiency of anti-cancer vaccination protocols.

It has been shown recently (Kammula, U. S., Marincola, F. M., and Rosenberg, S. A. (2000) Real-time quantitative polymerase chain reaction assessment of immune reactivity in melanoma patients after tumor peptide vaccination. J. Natl. Cancer Inst. 92: 1336-44) that the detection of cytokine mRNA associated to a short *in-vitro* stimulation (2 hours) of PBMC were able to detect epitope-specifiq CTLs in the PBMC's of patients undergoing vaccination with a tumor antigen. Nevertheless, according to the present invention this short *ex vivo* pulse is not essential.

### Example 4: Detection of the activation of the immune system of the recipient by the histocompatibility antigens of the donor.

In example 4, an organ (ex. liver, kidney, bone marrow, etc.) from a donor is transplanted to a recipient. Whole blood the recipient is collected in a tube comprising a compound inhibiting RNA degradation and/or gene induction according to present invention. RT-PCR is performed according to the method. Cytokine mRNA is measured as a read out of the activation of the immune system of the recipient by the histocompatibility antigens of the donor (Figure 1.3)

### Example 5: Detection of the reactivity of the immune system of the recipient to the histocompatibility antigens of the donor.

In example 5, an organ (ex. liver, kidney, bone marrow,...) from a donor is transplanted to a recipient. Whole blood of the recipient is collected on a tube and incubated *ex-vivo* with the histocompatibility antigens of the donor. A compound inhibiting RNA degradation and/or gene induction according to present invention is added to the blood. RT-PCR is performed according to the method. Cytokine mRNA is measured as a read out of the response of the immune system of the recipient by the histocompatibility antigens of the donor (Figure 1.4).

### Example of application: monitoring of rejection after organ transplantation

The monitoring of rejections of transplants is essentially based on the detection of markers measured in the urine or the blood of patients (blood urea nitrogen-BIN- or creatinine in the case of kidney transplants) or at the time of the analyses of biopsies of the grafted organ. These indicators are however only detected when the rejection mechanism is already well advanced. In fact, transplant rejection is the result of an immunological mechanism that precedes the deterioration of the grafted organ. The detection of these immunological mechanisms before the grafted organ is damaged would allow to reduce in a considerable manner the loss of the grafted organ by adapting more earlier the immunosuppressive treatments. On the other side, it is also recognized that of sub-clinical episodes of rejections (with no induction of clinical signs) occur themselves frequently after transplantation. These episodes sub-clinical rejection episodes could be the cause of chronic rejections. Several authors have investigate the detection of precocious immunologiques markers of organ rejection and particularly the detection in the circulation of recipient alloreactive T-lymphocytes directed against the allo-antigens of the donor. Methods include essentially the association of mixed cultures with the consecutive measurement of the proliferation of the lymphocytes of the receiver or the measurement of the production of cytokines by different methods (ELISA, ELISPOT, flow cytometry, etc.). More recently, other authors have looked on the characterization of lymphocytes activation markers patterns susceptible to underline precociously the triggering of a rejection mechanism. The detection of mRNA of genes expressed by the cytotoxic activated T-lymphocytes T activated (granzyme B, perforine, different cytokines) by sensitive methods of quantitative PCR were showed to be excellent tools to measure the triggering of a rejection. For this purpose, according to present invention, messengers coding for different kinds of cytokines may be studied, preferential targets may be IL-2, IFN-gamma, IL-4, IL-5, Granzyme, perforine and FasFas-ligand.

### Example 6: Immune monitoring in whole blood using real time PCR.

In example 6 a whole blood method is described allowing the measure of the induction of cytokine synthesis at the mRNA level. The originality of this method consists in the combination of PAXgene™ tubes containing a mRNA stabilizer for blood collection, the MagNA Pure™ instrument as an automated system for mRNA extraction and RT-PCR reagent mix preparation, and the real time PCR methodology on the Lightcycler™ for accurate and reproducible quantification of transcript levels. This example first demonstrate that this method is adequate to measure the induction of IL (interleukin)-1β and IL-1 receptor antagonist (IL-1 RA) mRNA upon addition of bacterial lipopolysaccharide (LPS) to whole blood. This example further demonstrates that this approach is also suitable to detect the production of mRNA encoding T cell-derived cytokines in whole blood incubated with tetanus toxoid as a model of *in vitro* immune response to a recall antigen. Finally, the example demonstrates that this methodology can be used successfully to assess inflammatory as well as T cell responses *in vivo,* as it allowed to detect the induction of IL-1β and IL-1 RA after injection of LPS in healthy volunteers, and also the induction of IL-2 upon recall immunisation with tetanus vaccine.

### MATERIAL AND METHODS.

***Blood collection for in vivo studies*.** For accurate quantification of peripheral blood mRNA levels, a 2.5-ml sample of blood was taken in a PAXgene™ tube for immediate cell lysis and nucleic acid precipitation. The mRNA is stable for up to 5 days in this blood lysate, the tubes being kept at room temperature until mRNA extraction.

***In vitro whole blood culture.** In vitro* whole blood LPS stimulation or tetanus toxoid rechallenge were performed on 200 µl of heparinized whole blood, and started at the latest four hours after blood collection. Cultures were stopped by adding 500 µl of the PAXgene™ tube's reagent, which induces total cell lysis and mRNA stabilisation. This allowed the use of the same mRNA extraction protocol for both *in vitro* and *in vivo* studies. ***mRNA extraction.*** The blood lysate obtained in the PAXgene™ tube or at the end of whole blood culture was briefly mixed before transferring a 300-µl aliquot in a 1.5-ml eppendorf tube for centrifugation at maximal speed for 5 minutes (12,000 to 16,000 g, depending on the device). The supernatant was discarded, and the nucleic acid pellet thoroughly dissolved by vortexing in 300 µl of the lysis buffer contained in the MagNA Pure™ mRNA extraction kit (Roche Applied Science). mRNA was then extracted from 300 µl of this solution, using this kit on the MagNA Pure™ instrument (Roche Applied Science) following manufacturer's instructions ("mRNA I I cells" Roche's protocol, final elution volume 100 µl). The quality of the extracted mRNA was previously documented by Northern blot analysis (Roche Applied Science, unpublished data).

***Real time PCR and reagent mix preparation**.* Reverse transcription and real time PCR were performed in one step, following the standard procedure described in the "Lightcycler™ - RNA Master Hybridisation Probes" Kit (Roche Applied Science). More precisely, the RT-PCR reaction was carried out in a 20 µl final volume containing: 1) H₂O up to 20 µl; 2) 7.5 µl RNA Master Hybridisation Probes 2.7x conc (RNA Master Hybridisation Probes Kit - Roche Applied Science); 3) 1.3 µl 50 mM Mn (OAc)₂; 4) 1, 2 or 3 µl of 6 pmoles/µl forward and reverse primers (final concentration 300, 600 or 900 nM, depending of the mRNA target; the conditions specific for each mRNA target are fully described in Stordeur et al, J Immunol Methods, 259 (1-2): 55-64, 2002, excepted for IL-2 and IL-4, which are listed in Table 4); 5) 1µl of 4 pmoles/µl TaqMan probe (final concentration 200 nM); 6) 5 µl purified mRNA or standard dilution. After an incubation period of 20 minutes at 61 °C to allow mRNA reverse transcription, and then an initial denaturation step at 95°C for 30 s, temperature cycling was initiated. Each cycle consisted of 95°C for 0 (zero) second and 60°C for 20 s, the fluorescence being read at the end of this second step (F1/F2 channels, no colour compensation). 45 cycles were performed, in total. All primers were chosen to span intronic sequences, so that genomic DNA amplification was not possible.
The RT-PCR reaction mixtures containing all reagents, oligonucleotides and samples, were fully prepared directly in the capillaries used on the Lightcycler™, by the MagNA Pure™ instrument. These capillaries were top closed, centrifuged and then introduced in the Lightcycler™ for one step RT-PCR. The sampling of all RT-PCR components was thus fully automated, avoiding manual sampling errors.

Results were expressed in copy numbers normalised against β-actin mRNA (mRNA copy numbers of cytokine mRNA per million of β-actin mRNA copies). For each sample, the mRNA copy number was calculated by the instrument software using the Ct value ("Arithmetic Fit point analysis") from a standard curve. This latter was constructed for each PCR run from serial dilutions of a purified DNA, as described in Stordeur et al, J Immunol Methods, 259 (1-2): 55-64, 2002.

***Experimental endotoxemia**.* Five healthy male volunteers (21-28 years) who had not taken any drugs for at least 10 days before the experiments were received an intravenous injection with a single dose of LPS (from *E.coli,* lot C; United States Pharmacopeial Convention, Rockville, MD; 4 ng/kg body weight). Ten minutes before, and 0.5, 1, 1.5, 2, 3 and 6 hours after the LPS injection, a 2.5 ml sample of blood was taken in a PAXgene™ tube. For *in vitro* studies, 200 µl of heparinized whole blood taken from healthy individuals were incubated with 10 ng/ml LPS (from *E.coli* serotype 0128:B12, Sigma-Aldrich, Bomem, Belgium) for 0 (beginning of the culture), 0.5, 1, 2 and 6 hours, at 37°C in a 5 % CO₂ atmosphere.

***Anti tetanus recall vaccination**.* Healthy volunteers (2 males, 4 females, 27-53 years) whom last tetanus toxoid vaccination was at least five years ago, received an intra muscular vaccine recall (Tevax, Smith Kline Beecham Biologicals, Rixensart, Belgium). A heparinized blood tube was taken the day of administration, 14 days before, and 3, 7, 14, 21 and 90 days after. 200 µl of blood were incubated, at 37°C in a 5 % CO₂ atmosphere, with or without 10 µg/ml tetanus toxoid (generous gift from Dr. E. Trannoy, Aventis Pasteur, Lyon, France) for 20 hours.

### RESULTS

***Measurement of IL-1β and IL-1 RA mRNA upon addition of bacterial LPS to whole blood.*** As demonstrated in Figure 5, addition of LPS (10 ng/ml) to whole blood led to a rapid induction of IL-1β and IL-1 RA mRNAs. This induction, already evident 30 to 60 minutes after LPS addition, resulted 6 hours after in a 47-fold and a 22-fold increase of the mRNA levels for IL-1β and IL-1 RA, respectively. The pattern of the curves suggests a rapid and sustained increase of both cytokine mRNAs amounts. In order to evaluate the accuracy of the system for mRNA quantification, the mRNA was quantified for β-actin and IL-1β from different volumes of LPS-stimulated whole blood, ranging from 20 to 200 µl. As shown in Figure 6, the mRNA copy numbers of both β-actin and IL-1β were indeed directly correlated with the starting volume of blood.

***In vitro response to tetanus toxoid**.* To determine whether this method might be suitable for the analysis of T cell responses, cytokine mRNA levels in whole blood culture after addition of tetanus toxoid, a well established recall antigen as all individuals were vaccinated in childhood, was quantified. A rapid and transient induction of IFN-γ, IL-2, IL-4 and IL-13 mRNA after incubation of whole blood with this antigen was found (Figure 7). When comparing the amplitude of the response for each cytokine, it appeared that the induction of IL-2 mRNA was the most pronounced. Indeed, the global increase of IL-2 mRNA copies after 16 hours of incubation in the presence of the toxoid was around 220 fold for the five independent experiments shown in Figure 7, while the maximum increase of IL-4 and IFN-γ mRNAs in the same experiments did not exceed 5 fold. Quantification of IL-2 mRNA therefore appears as the most sensitive parameters in this whole blood system assessing T cell responses. Data given in Table 5 indicates that the amplitude of the response to tetanus toxoid in this test is rather variable, probably depending on the moment of the last vaccine recall. The induction of IL-2 mRNA was effectively not observed after addition of tetanus toxoid to neonatal cord blood, indicating that only previously primed T cells and not naive T cells are able to respond in this assay (Table 5).

***Induction of IL-1 RA and IL-1β mRNA In whole blood after Intravenous Injection of LPS.*** As a first application of the method for the detection of cytokine induction *in vivo,* serial blood samples from healthy volunteers injected with a low dose (4 ng/kg) of bacterial lipopolysaccharide was analysed. A clear induction of both IL-1RA and IL-1β mRNA was observed (Figure 8). The induction of IL-1β mRNA was rapid, since it was already detected 30 to 60 minutes after endotoxin administration, and transient as IL-1β mRNA levels returned to pre-injection values after 6 hours. IL-1 RA mRNA was also induced, with a delayed kinetics as compared to IL-1β mRNA.

***Detection of anti-tetanus toxoid immune response after recall vaccination**.* As the *in vitro* experiments suggested that IL-2 mRNA was the most sensitive parameter to monitor anti-tetanus toxoid responses, this parameter was chosen to analyse the changes in the T cell responses to tetanus toxoid in whole blood upon recall vaccination *in vivo.* For this purpose, whole blood incubation in absence or presence of tetanus toxoid was performed before and at several time points after administration of the vaccine. As shown in Figure 9, the production of IL-2 mRNA in whole blood exposed to the antigen significantly increased in all vaccinated individuals. IL-2 mRNA induction was already apparent 7 days post vaccination, maximal levels being reached at day 14 or 21. The variability between individuals is probably related to differences in the basal status of anti-tetanus immunity (see also Table 5). The IL-2 response measured in whole blood after vaccination was specific for the immunising antigen as IL-2 mRNA levels measured in absence of *in vitro* restimulation were not significantly modified (Table 5).

### DISCUSSION

Real time PCR is so called because the amplicon accumulation can be directly monitored during the PCR process, using fluorogenic molecules that bind the PCR product. This leads to the generation of a fluorescence curve for each sample, from which it is possible to determine the (c)DNA copy number of the sample, by comparison to fluorescence curves obtained with calibrated standards. In order to enhance the specificity, the fluorogenic molecule can be an oligonucleotide complementary to a sequence of the PCR product, localised between the two primers. The new methodology, as described in the present application, provides a sensitive and accurate way to quantify nucleic acids in biological samples which was not possible using the prior art methods. The present application illustrates this by quantifying cytokine mRNA from purified cells or tissues representative of the *in vivo* situation.
One of the difficulties encountered using whole blood for RT-PCR analysis is the cell lysis that precedes RNA extraction. Because of the high amount of proteins present in plasma and erythrocytes, the majority of the methods that isolate RNA from whole blood involve the purification of the potential cellular sources of the analysed mRNA or the elimination of the red blood cells, before performing the RNA extraction. These intermediate steps can be associated with mRNA degradation and/or gene induction and thus with changes in mRNA levels. Furthermore, the simple fact of taking blood can lead to degradation of some mRNAs. This is especially true for cytokine mRNAs, which are sensitive to endogenous nucleases via the AU-rich sequences located in their 3' untranslated region. It was previously shown that peripheral blood IFN-γ mRNA levels indeed decreased by roughly 50 % already one hour after blood collection (Stordeur et al., (2002) J. Immunol Meth. 261:195). This can be avoided using quaternary amine surfactants such as tetradecyltrimethylammonium oxalate, a cationic surfactant called Catrimox-14™ (Qiagen, Westburg, Leusden, The Netherlands) that induces whole cell lysis and, in the same time, nucleic acid precipitation. The present example observes that the nucleic acid precipitate obtained with the PAXgene™ tubes can surprisingly be dissolved in a guanidium/ thiocyanate solution. An example of said solution is the lysis buffer provided with the MagNA Pure™ LC kits for mRNA isolation (Roche Applied Science). This prompted us to combine the use of PAXgene™ tubes with the MagNA Pure™ instrument, taking advantage of the high reproducibility and accuracy of the latter device due to the automated preparation of all of the components of the PCR reaction mixture.
Interestingly, the method of the present application was successfully applied to the detection of cytokine gene induction in whole blood upon endotoxin challenge *in vivo,* demonstrating that it could be used to monitor systemic inflammatory responses. The transient nature of the IL-1 response after *in vivo* challenge, contrasts with the persistent increase in IL-1 mRNA after *in vitro* addition of LPS to blood. This might be related to the rapid clearance of LPS *in vivo* but also to the redistribution of cytokine-producing cells *in vivo,* which is related to upregulation of adhesion molecules and chemokine receptors. Another possible application of this whole blood method is the monitoring of T cell responses upon vaccination, as suggested by the clear induction of IL-2 mRNA observed after *in vitro* rechallenge in individuals vaccinated with tetanus toxoid. This might be of special interest for large-scale vaccination studies in which cell isolation might be difficult to organise in good conditions, especially in developing countries where several new vaccines are under evaluation. To further investigate the applicability of this method in vaccine trials, it will be soon tested as read-out of T cell responses upon primary vaccination against hepatitis B.
The direct correlation between the starting volume of blood and the mRNA copy numbers (Figure 6) suggests that there is no absolute need to measure mRNA concentration for expression of the results using this method. However, because even small variations of the sample volume could result in quantification errors, it is preferable to correct the measured copies by simultaneous measurement of a housekeeping gene such as β-actin. This might still not be optimal as the expression of housekeeping genes might vary in certain conditions of stimulation. Therefore an external standard could be added to the sample before mRNA extraction. When the cellular source of a cytokine is well established such as in the case of T cells for IL-2, it might be appropriate to correct the numbers of cytokine gene copies by the numbers of copies encoding a gene specifically expressed in the corresponding cell type, such as CD3 in the latter example. Likewise, international standardisation of calibrators for cytokine mRNA quantification by real time PCR should be developed to facilitate comparison of data generated in different laboratories. Cytokine mRNA measurement in whole blood is useful for the monitoring of innate and adaptive immune responses required for the assessment of new vaccines and immunotherapies.

*Example* 7: *Automated mRNA extraction and reagent mix preparation on the MagNA Pure: direct correlation between amount of starting biological material and found copy number*.

The procedure followed in this example is summarized in Figure 10. In order to illustrate the accuracy of the system, a linear regression of mRNA copy number on starting cell number was calculated (Figure 11). mRNA was extracted from various peripheral blood mononuclear cell (PBMC) numbers (ranging from 100,000 to 600,000 cells, X-axis) and one step RT-real time PCR for β-actin mRNA was performed as described in the "Material and Methods" section of the present example 6. This experiment has been repeated from PBMC for β-actin and TNF-α mRNAs (Figure 12, panels B and D), and from whole blood (Figure 12, panel A) and CD4⁺ purified T cells (Figure 12, panel C) for β-actin mRNA.

### Example 8: Cancer immunotherapy

The procedure followed in this example is summarized in Figure 10. The methodology was applied to the monitoring of immune response induced by cancer vaccine. Figures 13, 14 and 15 illustrate the results obtained in this field with a melanoma patient.

### Example 9: Allergy

The procedure followed in this example is summarized in Figure 10. The methodology was then applied in Allergy. The response induced by *in vitro* incubation of whole blood of an allergic subject with the relevant allergen was analysed by IL-4 mRNA quantification using real-time PCR. Figures 16, 17, 18 and 19 illustrate the results obtained in this field.

### Example 10: Autoimmunity

The procedure followed in this example is summarized in Figure 10. The methodology was then applied in Autoimmunity. IL-2 mRNA quantification using this whole blood system was applied to assess T cell response to glutamic acid decarboxylase 65 (GAD65), an autoantigen being the target of auto-reactive T cells in type 1 autoimmune diabetes. Figures 20 and 21 illustrate the results obtained in this field.

### Example 11: Transplantation

The procedure followed in this example is summarized in Figure 10. The methodology was then applied in Transplantation. IL-2 mRNA quantification by real time PCR after whole blood incubation with alloreactive non-T cells provides an alternative to the classical mixed lymphocytes reaction (MLR) to monitor alloreactive T cell response. Figures 22 and 23 illustrate the results obtained in this field.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### References

Chomczynski, P. and Sacchi, N. (1987) Single-step method of RNA isolation by acid guanidinium thiocyanate-phenol-chloroform extraction. Analyt Biochem 162, 156.
Dahle, C.E. and Macfarlane, D.E. (1993) Isolation of RNA from cells in culture using Catrimox-14 cationic surfactant. Biotechniques 15, 1102.
Hartel, C., Bein, G., Muller-Steinhardt, M. and Kluter, H. (2001) Ex vivo induction of cytokine mRNA expression in human blood samples. J Immunol Methods 249, 63.
Pradier, O., Surquin, M., Stordeur, P., De Pauw, L., Kinnaert, P., Vereerstraeten, P., Capel, P., Goldman, M. and Abramowicz, D. (1996) Monocyte procoagulant activity induced by in vivo administration of the OKT3 monoclonal antibody. Blood 87, 3768.
Schmidt, W.N., Klinzman, D., LaBrecque, D.R., Macfarlane, D.E. and Stapleton, J.T. (1995) Direct detection of hepatitis C virus (HCV) RNA from whole blood, and comparison with HCV RNA in plasma and peripheral blood mononuclear cells. J Med Virol 47, 153.
Stordeur, P., Schandene, L., Durez, P., Gerard, C., Goldman, M. and Velu, T. (1995) Spontaneous and cycloheximide-induced interleukin-10 mRNA expression in human mononuclear cells. Mol.Immunol. 32, 233.
Stordeur, P., Poulin, L.F., Craciun, L., Zhou, L., Schandené, L., de Lavareille, A., Goriely, S. and Goldman, M. Cytokine mRNA Quantification by Real Time PCR. J Immunol Methods, 259 (1-2): 55-64, 2002.

**TABLE 1. Oligonucleotides for real time PCR as used in Stordeur et al, J Immunol Methods, 259 (1-2): 55-64, 2002.**

| **mRNA targets** | **Oligonucleotides (5'→3') *** | **Product size (bp)** | **Final concentration (nM) **** |
|---|---|---|---|
| IL-1ra | F264 : GAAGATGTGCCTGTCCTGTGT | 80 | F 900 R 900 |
| | R343 : CGCTCAGGTCAGTGATGTTAA | | |
| | P291 : 6Fam-TGGTGATGAGACCAGACTCCAGCTG-Tamra-p | | |
| IL-1β | F176 : ACAGATGAAGTGCTCCTTCCA | 73 | F 600 R 900 |
| | R248 : GTCGGAGATTCGTAGCTGGAT | | |
| | P207 : 6Fam-CTCTGCCCTCTGGATGGCGG-Tamra-p | | |
| IL-5 | F83 : AGCTGCCTACGTGTATGCCA | 71 | F 300 R 900 |
| | R153 : GCAGTGCCAAGGTCTCTTTCA | | |
| | P104: 6Fam-CCCCACAGAAATTCCCACAAGTGCATT-Tamra-p | | |
| IL-10 | F409 : CATCGATTTCTTCCCTGTGAA | 74 | F 600 R 900 |
| | R482 : TCTTGGAGCTTATTAAAGGCATTC | | |
| | P431: 6Fam-ACAAGAGCAAGGCCGTGGAGCA-Tamra-p | | |
| IL-13 | F155 : TGAGGAGCTGGTCAACATCA | 76 | F 900 R 900 |
| | R230 : CAGGTTGATGCTCCATACCAT | | |
| | P187 : 6Fam-AGGCTCCGCTCTGCAATGGC-Tamra-p | | |
| TNF-α | F275 : CCCAGGGACCTCTCTCTAATC | 84 | F 900 R 900 |
| | R38 : ATGGGCTACAGGCTTGTCACT | | |
| | P303 : 6Fam-TGGCCCAGGCAGTCAGATCATC-Tamra-p | | |
| IFN-γ | F464 : CTAATTATICGGTAACTGACTTGA | 75 | F 600 R 900 |
| | R538 : ACAGTTCAGCCATCACTTGGA | | |
| | P491 : 6Fam-TCCAACGCAAAGCAATACATGAAC-Tamra-p | | |
| β-actin | F976 : GGATGCAGAAGGAGATCACTG | 90 *** | F 300 R 300 |
| | R1065 : CGATCCACACGGAGTACTTG | | |
| | P997 : 6Fam-CCCTGGCACCCAGCACAATG-Tamra-p | | |
| Mouse IL-9 (TaqMan probe) | F91: GGCATCAGAGACACCAATTACCT R233 : TGGCATTGGTCAGCTGTAACA P184:6Fam-CTCTCCGTCCCAACTGATGATTGTACCAC-Tamra-p | 143 | F 300 R 300 |
| Mouse IL-9 (hybridisation probes) | F91: GGCATCAGAGACACCAATTACCT R233 : TGGCATTGGTCAGCTGTAACA P163 : AACGTGACCAGCTGCTTGTGT-fluorescein P185 : LCred 640-TCTCCGTCCCAACTGATGATT-p | 143 | F 300 R 900 |

| | | | |
|---|---|---|---|
| * F, R and P indicate forward and reverse primers and probes, respectively; numbers indicate the sequence position. * Final concentration of forward (F) and reverse (R) primers. *** Except for IL-5, all primers were chosen to span intronic sequences so that genomic DNA amplification is not possible, excepted for β-actin for which a 112 bp longer band is obtained. If contaminating genomic DNA is detected using this size difference on agarose gel, a DNase digestion of all of the RNA samples coming from the same experiment is performed. | | | |

**TABLE 2. Oligonucleotides for standard preparation. Stordeur et al, J Immunol Methods, 259 (1-2): 55-64, 2002.**

| **mRNA targets** | **Oligonucleotides (5'→3') *** | **Product size (bp)** | **Conditions for "classical" PCR **** |
|---|---|---|---|
| IL-1ra | F43 :CTCCTCTTCCTGTTCCATTC R493 : CTTCGTCAGGCATATTGGT | 451 | **A = 56 Mg = 1.5** |
| IL-1β | F59 : CTTCATTGCTCAAGTGTCTGAA R553 : ACTTGTTGCTCCATATCCTGTC | 495 | A = 58 Mg = 1.5 |
| IL-10 | F296 : TTTACCTGGAGGAGGTGATG R771 : TTGGGCTTCTTTCTAAATCGT | 476 | A = 56 Mg = 1.5 |
| IL-13 | F23 : GCTCCTCAATCCTCTCCTGT R507 : GCAACTTCAATAGTCAGGTCCT | 485 | A = 56 Mg = 1.0 |
| TNF-α | F83 : ACCATGAGCACTGAAAGCAT R488 : AGATGAGGTACAGGCCCTCT | 406 | A = 58 Mg = 1.5 |
| IFN-γ | F154 : TTGGGTTCTCTTGGCTGTTA R632 : AAATATTGCAGGCAGGACAA | 479 | A = 58 Mg = 1.5 |
| β-actin | F745 : CCCTGGAGAAGAGCTACGA R1253 : TAAAGCCATGCCAATCTCAT | 509 | A = 58 Mg = 1.5 |

| | | | |
|---|---|---|---|
| * F and R indicate forward and reverse primers, respectively; numbers indicate the sequence position. ** Conditions, for all targets, were as follows: denaturation at 95 °C for 20 s, annealing (temperature as stated (A)) for 20 s and elongation at 72°C for 45 s, for a total of 35 cycles. MgCl₂ concentration (Mg, mM) was as stated. For the complete procedure see (Stordeur et al., (1995), PCR for IFN-γ). | | | |

**TABLE 3. Comparison of Qiagen and MagNA Pure LC extraction methods.**

| **3.1. Qiagen mRNA extraction method. Blood mRNA coming from the same blood sample was extracted 9 times.** | |
|---|---|
| | **IFN-γ mRNA copy numbers per million of β-actin mRNA copies** |
| result 1 | 35 |
| result 2 | 25 |
| result 3 | 29 |
| result 4 | 27 |
| result 5 | 27 |
| result 6 | 49 |
| result 7 | 33 |
| result 8 | 22 |
| result 9 | 27 |
| | |
| ***mean*** | ***30*** |
| ***SD*** | ***8*** |
| ***CV*** | ***26*** |

| **3.2. MagNA Pure LC (kit + instrument) mRNA extraction method. Blood mRNA prepared from the same blood sample was extracted 9 times.** | |
|---|---|
| | **IFN-γ mRNA copy numbers per million of β-actin mRNA copies** |
| result 1 | 192 |
| result 2 | 170 |
| result 3 | 153 |
| result 4 | 139 |
| result 5 | 138 |
| result 6 | 160 |
| result 7 | 105 |
| result 8 | 142 |
| result 9 | 142 |
| | |
| ***mean*** | ***149*** |
| ***SD*** | ***24*** |
| ***CV*** | ***16*** |

**Table 4. Oligonucleotides for (real time) PCR¹**

| **PRIMERS AND PROBES FOR REAL TIME PCR** | | | | | |
|---|---|---|---|---|---|
| **mRNA target** | **Oligonucleotides (5'→3') ²** | | **Product size (bp)** | | **Final concentration (nM) ³** |
| IL-2 | F273: CTCACCAGGATGCTCACATTTA R367: TCCAGAGGTTTGAGTTCTTCTTCT P304: 6Fam-TGCCCAAGAAGGCCACAGAACTG-Tamra-p | | 95 | | F 900 R900 |
| IL-4 | F174: ACTTTGAACAGCCTCACAGAG R247: TTGGAGGCAGCAAAGATGTC P204: 6Fam-CTGTGCACCGAGTTGACCGTA-Tamra-p | | 74 | | F 300 R 900 |
| **PRIMERS FOR STANDARD PREPARATION BY "CLASSICAL" PCR ⁴** | | | | | |
| **mRNA target** | | **Oligonucleotides (5'→3') ²** | | **Product size (bp)** | |
| IL-2 | | F155: TGTCACAAACAGTGCACCTACT R672: AGTTACAATAGGTAGCAAACCATACA | | 518 | |
| IL-4 | | F27: TAATTGCCTCACATTGTCACT R529: ATTCAGCTCGAACACTTTGAA | | 503 | |

| | | | | | |
|---|---|---|---|---|---|
| 1. For a full description, see Stordeur et al, J Immunol Methods, 259 (1-2): 55-64, 2002. 2. F, R and P indicate forward and reverse primers and probes, respectively; numbers indicate the sequence position from Genebank accession numbers X01586 for IL-2 and NM_000589 for IL-4. 3. Final concentration of forward (F) and reverse (R) primers. 4. Standard curves were generated from serial dilutions of PCR products prepared by "classical" PCR, for which specific conditions were as follows: denaturation at 95°C for 20 s, annealing at 58°C for 20 s and elongation at 72°C for 45 s, for a total of 35 cycles. MgCl₂ final concentration was 1.5 mM. | | | | | |

**Table 5.**

| **Tetanus** | Cord blood | **Adult whole blood** |
|---|---|---|
| **Toxoid** | | **(before vaccine recall)** |
| -- | 109 ± 51 | 1,154 ± 1,194 |
| + | 159 ± 91 | 7,715 ± 8,513 |

## Claims

1. A method for the quantification of real *in vivo* levels of low-level or unstable RNA from whole blood comprising the steps of:
(a) collecting the whole blood in a tube comprising a compound inhibiting RNA degradation and/or gene induction,
(b) forming a precipitate comprising nucleic acids,
(c) separating said precipitate of step (b) from the supernatant,
(d) dissolving said precipitate of step (c) using a buffer, forming a suspension,
(e) isolating nucleic acids from said suspension of step (d) using an automated device,
(f) dispersing/distributing a reagent mix for RT-PCR using an automated device,
(g) dispersing/distributing the nucleic acids isolated in step (e) within the dispersed reagent mix of step (f) using an automated device, and,
(h) determining the real *in vivo* levels of low-level or unstable RNA using the nucleic acid/RT-PCR reagent mix of step (g) in an automated setup.

2. The method according to claim 1, wherein steps (a) and (b) are performed simultaneously.

3. The method according to claim 1 or 2, wherein the compound of step (a) comprises a quaternary amine surfactant.

4. The method according to claim 3, wherein said quaternary amine is tetradecyltrimethyl-ammonium oxalate.

5. The method according to any of the claims 1 to 4, wherein the tube of step (a) is an open or a closed blood collecting tube.

6. The method according to any of the claims 1 to 5, wherein the buffer of step (d) is a guanidine-thiocyanate-containing buffer.

7. The method according to any of the claims 1 to 6, wherein the isolation of nucleic acids of step (e) is performed using RNA-capturing beads.

8. The method according to any of the claims 1 to 7, wherein said real *in vivo* levels are determined using real time PCR.

9. The method according to any of the claims 1 to 8, wherein said quantification is performed using a biological sample of 20-200 µl.

10. The method according to any of the claims 1 to 8, wherein said quantification is performed using a biological sample of 100 µl.

11. A kit for the quantification of real *in vivo* levels of low-level or unstable RNA from whole blood comprising:
(a) a compound inhibiting RNA degradation and/or gene induction,
(b) an RNA precipitate dissolving buffer,
(c) reagents for automated RNA isolation,
(e) a reagent mix for a simultaneous RT and real-time PCR reaction or separate compounds thereof , allowing the automated dispersion of said mix, and,
(e) an instruction manual describing a method for an automated RNA isolation from whole blood, a method for the automated dispersion of a reagent mix and the dispersion of the isolated nucleic acids for RT- real time PCR, and a method for automated RNA analysis to quantify real in vivo levels of low-level or unstable RNA.

12. The kit according to claim 11, wherein the compound of part (b) is a compound as defined in the methods of claims 3 or 4.

13. The kit according to claims 11 and 12, wherein the RNA precipitate dissolving buffer is a buffer as defined in the method of claim 6.

## Patentansprüche

1. Verfahren zur Quantifizierung von realen *In-vivo-*Spiegeln von niedrigen RNA-Spiegeln oder instabiler RNA aus Vollblut, das die folgenden Schritte umfasst:
(a) Sammeln des Vollbluts in einem Röhrchen, das eine Verbindung umfasst, die den RNA-Abbau und/oder die Geninduktion inhibiert,
(b) Bilden eines Präzipitats, das Nukleinsäuren umfasst,
(c) Abscheiden des Präzipitats aus Schritt (b) von dem Überstand,
(d) Auflösen des Präzipitats aus Schritt (c) unter Verwendung eines Puffers, wobei eine Suspension gebildet wird,
(e) Isolieren der Nukleinsäuren aus der Suspension aus Schritt (d) unter Verwendung einer automatisierten Vorrichtung,
(f) Dispergieren/Verteilen eines Reagensgemischs zur RT-PCR unter Verwendung einer automatisierten Vorrichtung,
(g) Dispergieren/Verteilen der Nukleinsäuren, die in Schritt (e) isoliert wurden, innerhalb des dispergierten Reagensgemischs aus Schritt (f) unter Verwendung einer automatisierten Vorrichtung, und,
(h) Bestimmen der realen *In*-*vivo*-Spiegel von niedrigen RNA-Spiegeln oder instabiler RNA unter Verwendung des Nukleinsäure/RT-PCR-Reagensgemischs aus Schritt (g) in einer automatisieren Apparatur.

2. Verfahren nach Anspruch 1, wobei die Schritte (a) und (b) gleichzeitig ausgeführt werden.

3. Verfahren nach Anspruch 1 oder 2, wobei die Verbindung aus Schritt (a) ein quaternäres Amintensid umfasst.

4. Verfahren nach Anspruch 3, wobei das quaternäre Amin Tetradecyltrimethylammoniumoxalat ist.

5. Verfahren nach einem der Ansprüche 1 bis 4, wobei das Röhrchen aus Schritt (a) ein offenes oder geschlossenes Blutentnahmeröhrchen ist.

6. Verfahren nach einem der Ansprüche 1 bis 5, wobei der Puffer aus Schritt (d) ein Puffer ist, der Guanidinthiocyanat enthält.

7. Verfahren nach einem der Ansprüche 1 bis 6, wobei die Isolierung der Nukleinsäuren aus Schritt (e) unter Verwendung von Kugeln zum RNA-Einfang ausgeführt wird.

8. Verfahren nach einem der Ansprüche 1 bis 7, wobei die realen *In-vivo*-Spiegel unter Verwendung von Echtzeit-PCR bestimmt werden.

9. Verfahren nach einem der Ansprüche 1 bis 8, wobei die Quantifizierung unter Verwendung einer biologischen Probe von 20 bis 200 µl ausgeführt wird.

10. Verfahren nach einem der Ansprüche 1 bis 8, wobei die Quantifizierung unter Verwendung einer biologischen Probe von 100 µl ausgeführt wird.

11. Kit zur Quantifizierung von realen *In-vivo*-Spiegeln von niedrigen RNA-Spiegeln oder instabiler RNA aus Vollblut, der Folgendes umfasst:
(a) eine Verbindung, die RNA-Abbau und/oder Geninduktion inhibiert,
(b) einen Puffer, der das RNA-Präzipitat auflöst,
(c) Reagenzien zur automatisierten RNA-Isolierung,
(e) ein Reagensgemisch zur gleichzeitigen RT- und Echtzeit-PCR-Reaktion oder getrennte Verbindungen davon, die die automatisierte Dispersion des Gemischs ermöglichen, und
(e) eine Bedienungsanleitung, die ein Verfahren zur automatisierten RNA-Isolierung aus Vollblut, ein Verfahren zur automatisierten Dispersion eines Reagensgemischs und die Dispersion der isolierten Nukleinsäuren für RT-Echtzeit-PCR und ein Verfahren zur automatisierten RNA-Analyse umfasst, um reale In-vivo-Spiegel von niedrigen RNA-Spiegeln oder instabiler RNA zu quantifizieren.

12. Kit nach Anspruch 11, wobei die Verbindung von Teil (b) eine Verbindung ist, wie in den Verfahren der Ansprüche 3 oder 4 definiert.

13. Kit nach den Ansprüchen 11 und 12, wobei der Puffer, der das RNA-Präzipitat auflöst, ein Puffer ist, wie in dem Verfahren nach Anspruch 6 definiert.

## Revendications

1. Procédé pour la quantification de taux réels *in vivo* d'ARN de bas niveau ou instable à partir de sang total comprenant les étapes consistant à :
(a) recueillir le sang total dans un tube comprenant un composé inhibant la dégradation de l'ARN et/ou l'induction de gènes,
(b) former un précipité comprenant des acides nucléiques,
(c) séparer ledit précipité de l'étape (b) du surnageant,
(d) dissoudre ledit précipité de l'étape (c) à l'aide d'un tampon, former une suspension,
(e) isoler les acides nucléiques de ladite suspension de l'étape (d) en utilisant un dispositif automatisé,
(f) disperser/distribuer un mélange réactionnel pour la méthode RT-PCR en utilisant un dispositif automatisé,
(g) disperser/distribuer les acides nucléiques isolés à l'étape (e) dans le mélange réactionnel dispersé de l'étape (f) en utilisant un dispositif automatisé, et
(h) déterminer les taux réels *in vivo* d'ARN de bas niveau ou instable en utilisant les acides nucléiques/le mélange réactionnel RT-PCR de l'étape (g) dans une configuration automatisée.

2. Procédé selon la revendication 1, dans lequel les étapes (a) et (b) sont réalisées simultanément.

3. Procédé selon la revendication 1 ou la revendication 2, dans lequel le composé de l'étape (a) comprend un agent tensioactif amine quaternaire

4. Procédé selon la revendication 3, dans lequel ladite amine quaternaire est l'oxalate de tétradécyltriméthylammonium.

5. Procédé selon l'une quelconque des revendications 1 à 4, dans lequel le tube de l'étape (a) est un tube pour recueillir du sang ouvert ou fermé.

6. Procédé selon l'une quelconque des revendications 1 à 5, dans lequel le tampon de l'étape (d) est un tampon contenant de la guanidine et du thiocyanate.

7. Procédé selon l'une quelconque des revendications 1 à 6, dans lequel l'isolation des acides nucléiques de l'étape (e) est réalisée en utilisant des billes de capture d'ARN.

8. Procédé selon l'une quelconque des revendications 1 à 7, dans lequel lesdits taux réels *in vivo* sont déterminés en utilisant une PCR en temps réel.

9. Procédé selon l'une quelconque des revendications 1 à 8, dans lequel ladite quantification est réalisée en utilisant un échantillon biologique de 20 - 200 µl.

10. Procédé selon l'une quelconque des revendications 1 à 8, dans lequel ladite quantification est réalisée en utilisant un échantillon biologique de 100 µl.

11. Kit pour la quantification de taux réels *in vivo* d'ARN de bas niveau ou instable à partir de sang total comprenant :
(a) un composé inhibant la dégénération d'ARN et/ou l'induction de gènes,
(b) un tampon de dissolution de précipité d'ARN,
(c) des réactifs pour une isolation de l'ARN automatisée,
(d) un mélange réactionnel pour une réaction RT et PCR en temps réel simultanée ou des composés séparés de celles-ci, permettant la dispersion automatisée dudit mélange, et
(e) un manuel d'instructions décrivant un procédé pour une isolation automatisée de l'ARN du sang total, un procédé pour la dispersion automatisée d'un mélange réactionnel et la dispersion des acides nucléiques isolés pour une RT-PCR en temps réel, et un procédé pour une analyse automatisée de l'ARN pour quantifier les taux réels *in vivo* d'ARN de bas niveau ou instable.

12. Kit selon la revendication 11, dans lequel le composé de la partie (b) est un composé tel que défini dans les procédés des revendications 3 ou 4.

13. Kit selon les revendications 11 et 12, dans lequel le tampon de dissolution de précipité d'ARN est un tampon tel que défini dans le procédé de la revendication 6.
